# EUROPEAN PATENT SPECIFICATION

(11) **EP 1 487 424 B1**
(45) Date of publication and mention of the grant of the patent: **13.09.2006**
(21) Application number: 03727265.5
(22) Date of filing: 14.03.2003
(51) Int. Cl.: A61K 31/00, A61K 31/506, A61K 31/41

(54) **4-(4-METHYLPIPERAZIN-1-YLMETHYL)-N-(4-METHYL-3(4-PYRIDIN-3-YL)PYRIMIDIN-2-YL-AMINO)PHENYL)-BENZAMIDE FOR TREATING ANG II-MEDIATED DISEASES**
4-(4-METHYLPIPERAZIN-1-YLMETHYL)-N-(4-METHYL-3-(4-PYRIMINDIN-3-YL)PYRIMIDIN-2-YL-AMINO)PHENYL)-BENZAMIDE ZUR BEHANDLUNG VON ANG-II VERMITTELT KRANKHEITEN
4-(4-METHYLPIPERAZIN-1-YLMETHYL)-N-(4-METHYL-3(4-PYRIDIN-3-YL)PYRIMIDIN-2-YL-AMINO)PHENYL)-BENZAMIDE POUR LE TRAITEMENT DE MALADIES INDUITES PAR L'ANGIOTENSINE II

(30) Priority: 15.03.2002 GB 0206216; 15.03.2002 GB 0206217; 29.07.2002 GB 0217505
(43) Date of publication of application: 22.12.2004
(73) Proprietor: Novartis AG, 4056 Basel (CH); The University of Melbourne, Victoria 3010 (AU)
(72) Inventor: GILBERT, Richard, Ernest, West St. Kilda, VIC 3182 (AU); KELLY, Darren, James, Wonga Park, VIC 3115 (AU); FELDMAN, David, Louis, Teaneck, NJ 07666 (US)
(74) Representative: de Weerd, Petrus G.W.
(86) International application number: PCT/EP2003/002709
(87) International publication number: WO 2003/077892

(56) References cited:
- WO-A-99/03854

## Description

The present invention relates to a new use of PDGF receptor tyrosine kinase inhibitors, especially of N-phenyl-2-pyrimidine-amine derivatives of formula I (hereinafter; COMPOUNDS I) in which the symbols and substituents have the meaning as given hereinafter in free form or in pharmaceutically acceptable salt form, said compound groups being referred to hereinafter collectively as COMPOUNDS OF THE INVENTION, for the manufacture of pharmaceutical compositions for the treatment specified of Ang II-mediated diseases, to a method of treating warm-blooded animals including humans suffering from Ang II-mediated diseases by administering to a said animal in need of such treatment an effective dose of at least one COMPOUND OF THE INVENTION or a pharmaceutically acceptable salt thereof, and to a combination which comprises (a) a PDGF receptor tyrosine kinase inhibitor preferably N-{5-[4-(4-methyl-piperazino-methyl)-benzoylamido]-2-methylphenyl}-4-(3-pyridyl)-2-pyrimidine-amine (designated hereinafter as COMPOUND I) and (b) at least one compound selected from an antihypertensive, an aldosterone antagonist, an aldosterone synthase inhibitor and/or an angiotensin receptor blocker agent and optionally at least one pharmaceutically acceptable carrier for simultaneous, separate or sequential use, in particular for the treatment of hypertension and hypertension-induced diseases.

The renin-angiotensin system (RAS) upregulation is a well-recognized contributor to the pathogenesis of cardiovascular disease and is one of the hormonal mechanisms involved in regulation of pressure/volume homeostasis and in expression of hypertension. The renin-angiotensin system furthermore plays a major role in the development of renal damage. The inhibition of the renin-angiotensin system has been shown to produce a beneficial effect on the kidney. In addition to the hemodynamic effects, Ang II is known to produce direct effects, such as stimulatory actions on mesangial cell growth and the production of extracellular matrix. Together with the presence of a local renin-angiotensin system in renal glomeruli, Ang II may directly modulate renal structure, which may lead to renal damage and diseases(i.e. renal Hyperfiltration such as after portal renal ablation, proteinuria in chronic renal disease, renal arteriopathy as a consequence of hypertension, Nephrosclerosis or hypertensive nephrosclerosis) (Keiko *Matsumoto* et Al. Hypertension. *1999;34:279-284).* While initially viewed as a purely vasoactive hormone, evidence accumulated over more than a decade suggests that angiotensin II, the dominant effector molecule of the renin-angiotensin system (RAS), may also act as a growth factor in responsive tissues, such as those of the cardiovascular system. These growth factor-like effects include vascular smooth muscle hypertrophy (Geisterfer AA, Peach MJ, Owens GK: Angiotensin II induces hypertrophy, not hyperplasia, of cultured rat aortic smooth muscle cells. *Circ Res* 62:749-756., 1988) and the synthesis of extracellular matrix, together contributing to angiotensin II-induced hypertrophic vascular remodeling. The same is true for angiotensin II induced cardiac remodeling. The mechanisms whereby angiotensin II mediates these growth factor-like effects and their separation from secondary hemodynamic changes are incompletely understood but may involve multiple mechanisms. For instance, experimental studies indicate that angiotensin II stimulates the expression of transforming growth factor-β (TGF-β), a potent inducer of both matrix synthesis and cell hypertrophy. Hypertension affects the vasculature of all major organ systems (e.g., heart, brain, kidneys), and myocardial infarction and congestive heart failure (CHF) account for the majority of deaths secondary to hypertension (i.e., hypertension is a major etiologic factor in development of CHF). It seems that hypertension is associated with hypertrophy of the vascular smooth muscle cell and accumulation of the extracellular matrix (vessel remodeling). This hypertrophic, not hyperplastic, smooth muscle cell adaptation is found in many models of hypertension. Finally, Parker SB (Hypertension 1998 Sep;32(3):452-8) showed that (1) arterial hypertrophy from Ang II infusion occurs in response to elevated arterial pressure, (2) hypertrophy was not associated with hyperplasia or polyploidy of vascular smooth muscle cells, and (3) PDGF-A expression correlated with elevated pressure and arterial wall hypertrophy.
Vascular damage related to heart failure and myocardial infarction correlate with the renin-angiotensin system activation.
Recent observations point to an upregulation of the local renin-angiotensin system (RAS) and the synthesis of angiotensin II (Ang II) as critical factors in the activation of myocyte apoptosis and reactive hypertrophy in a model of insulin-dependent diabetes (Andrea Frustaci and al.; Circulation Research (2000); 87:1123-1132).
The localization of Ang II increased in myocytes and capillary endothelial cells of diabetic and diabetic-hypertensive hearts. Clinical and experimental results indicate that diabetes is characterized by upregulation of the systemic and local RAS, and interventions attenuating the effects of Ang II positively interfere with cardiovascular complications such as described herein (diseases involving angiotensin II induced hypertrophy or angiotensin II induced hypertrophic remodeling in the cardiovascular system) and more specifically diabetic congestive heart failure, myopathy, cardiomyopathy or myocardial hypertrophy. Furthermore, severe hypertension involves stimulation of the systemic and local RAS, myocyte loss, and cellular hypertrophy, suggesting that diabetes and hypertension together may synergistically damage the structure and function of the overloaded human heart (Hypertension may enhance Ang II formation, potentiating the cardiac damage). Upregulation of Intercellular adhesion molecule-1 (ICAM-1) by Ang II is involved in increasing the recruitment of leukocytes, followed by adhesion and transmigration, resulting in further endothelial cell damage and microvascular dysfunction. Hence, early increased expression of ICAM-1 caused by Ang II may be an important initiating event in target organ damage in diseases characterized by activation of the renin-angiotensin system. Furthermore, it has been suggested that rises in Ang II independent of elevations in blood pressure may initiate damage. Thus, chronic damage to the endothelium could lead to decreased nitric oxide bioavailability and, hence, increased peripheral vascular resistance, hypertension, and left or right ventricular hypertrophy. When circulating Ang II is elevated in these diseases, vascular damage may ensue due to Ang II-induced overexpression of ICAM-1 as a result of enhanced leukocyte binding. This increased binding could alter vascular function and either directly or indirectly promote damage to the heart. Direct injury to the heart may occur through the infiltration of monocytes into cardiac muscle. Indeed, a recent study showed that monocyte binding to ICAM-1 leads to reduced contractility of cardiac myocytes and suggested that this damage is mediated by reactive oxygen species. Indirect injury may be related to vascular endothelial dysfunction, which reportedly arises from monocyte binding to the endothelium associated with an increase in superoxide anion. Thus, chronic damage to the endothelium could lead to decreased nitric oxide bioavailability and, hence, increased peripheral vascular resistance, hypertension, and left or right ventricular hypertrophy. Right ventricular myocardial hypertrophy occurs also during hypoxic pulmonary hypertension which is associated with local renin-angiotensin system activation. Alternatively, endothelial dysfunction may lead to reduced perfusion of the cardiac muscle, which in tum causes myocardial infarction. Angiotensin II is a potent mediator of oxidative stress and stimulates the release of cytokines and the expression of leukocyte adhesion molecules that mediate vessel wall inflammation. Thus, Ang II is deeply involved in endothelial dysfunction with pro-inflammatory and pro-oxidant states.

Pursuing research in this field, the applicant has surprisingly discovered that PDGF receptor tyrosine kinase inhibitor especially COMPOUNDS I attenuate the Ang II-mediated injuries. The COMPOUNDS OF THE INVENTION reduce particularly the matrix deposition and vascular hypertrophy and hypertrophic remodeling in animals. Secondly, the amelioration of these trophic effects was independent of changes in systemic blood pressure. The advantage of a blood pressure independent effect is that in clinical practice it is difficult to reach recommended targets. It usually requires 2-3 drugs. Also, there is no threshold for blood pressure or angiotensin II induced injuries including heart failure, so a blood pressure independent protection would be highly advantageous.

The present invention thus concerns the use of a PDGF receptor tyrosine kinase inhibitor especially of N-phenyl-2-pyrimidine-amine derivatives of formula I or pharmaceutically acceptable salt thereof, for the manufacture of a medicament for treating specified Ang II-mediated diseases.

In particular, the present invention relates to a new use of N-phenyl-2-pyrimidine-amine derivatives of formula I, 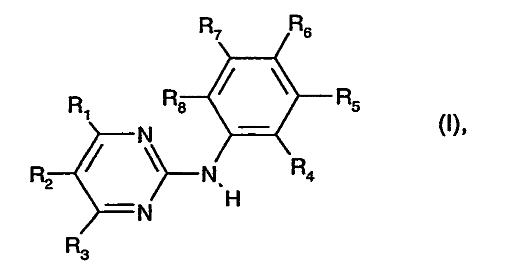 wherein
R₁ is 4-pyrazinyl; 1-methyl-1 H-pyrrolyl; amino- or amino-lower alkyl-substituted phenyl, wherein the amino group in each case is free, alkylated or acylated; 1 H-indolyl or 1H-imidazolyl bonded at a five-membered ring carbon atom; or unsubstituted or lower alkylsubstituted pyridyl bonded at a ring carbon atom and unsubstituted or substituted at the nitrogen atom by oxygen;
R₂ and R₃ are each independently of the other hydrogen or lower alkyl;
one or two of the radicals R₄, R₅, R₆, R₇ and R₈ are each nitro, fluoro-substituted lower alkoxy or a radical of formula II

-N(R₉)-C(=X)-(Y)ₙ-R₁₀ (II),

wherein
R₉ is hydrogen or lower alkyl,
X is oxo, thio, imino, N-lower alkyl-imino, hydroximino or O-lower alkyl-hydroximino,
Y is oxygen or the group NH,
n is 0 or 1 and
R₁₀ is an aliphatic radical having at least 5 carbon atoms, or an aromatic, aromaticaliphatic, cycloaliphatic, cycloaliphatic-aliphatic, heterocyclic or heterocyclic-aliphatic radical,
and the remaining radicals R₄, R₅, R₆, R₇ and R₈ are each independently of the others hydrogen, lower alkyl that is unsubstituted or substituted by free or alkylated amino, piperazinyl, piperidinyl, pyrrolidinyl or by morpholinyl, or lower alkanoyl, trifluoromethyl, free, etherified or esterifed hydroxy, free, alkylated or acylated amino or free or esterified carboxy,
or of a salt of such a compound having at least one salt-forming group,
for the manufacture of a medicament for treating Ang II-mediated diseases.

1-Methyl-1H-pyrrolyl is preferably 1-methyl-1H-pyrrol-2-yl or 1-methyl-1H-pyrrol-3-yl.

Amino- or amino-lower alkyl-substituted phenyl R₁ wherein the amino group in each case is free, alkylated or acylated is phenyl substituted in any desired position (ortho, meta or para) wherein an alkylated amino group is preferably mono- or di-lower alkylamino, for example dimethylamino, and the lower alkyl moiety of amino-lower alkyl is preferably linear C₁-C₃alkyl, such as especially methyl or ethyl.

1H-Indolyl bonded at a carbon atom of the five-membered ring is 1H-indol-2-yl or 1H-indol-3-yl.

Unsubstituted or lower alkyl-substituted pyridyl bonded at a ring carbon atom is lower alkyl-substituted or preferably unsubstituted 2-, 4- or preferably 3-pyridyl, for example 3-pyridyl, 2-methyl-3-pyridyl or 4-methyl-3-pyridyl. Pyridyl substituted at the nitrogen atom by oxygen is a radical derived from pyridine N-oxide, i.e. N-oxido-pyridyl.

Fluoro-substituted lower alkoxy is lower alkoxy carrying at least one, but preferably several, fluoro substituents, especially trifluoromethoxy or 1,1,2,2-tetrafluoro-ethoxy.

When X is oxo, thio, imino, N-lower alkyl-imino, hydroximino or O-lower alkyl-hydroximino, the group C=X is, in the above order, a radical C=O, C=S, C=N-H, C=N-lower alkyl, C=N-OH or C=N-O-lower alkyl, respectively. X is preferably oxo.

n is preferably 0, i.e. the group Y is not present.

Y, if present, is preferably the group NH.

The term "lower" within the scope of this text denotes radicals having up to and including 7, preferably up to and including 4 carbon atoms.

Lower alkyl R₁, R₂, R₃ and R₉ is preferably methyl or ethyl.

An aliphatic radical R₁₀ having at least 5 carbon atoms preferably has not more than 22 carbon atoms, generally not more than 10 carbon atoms, and is such a substituted or preferably unsubstituted aliphatic hydrocarbon radical, that is to say such a substituted or preferably unsubstituted alkynyl, alkenyl or preferably alkyl radical, such as C₅-C₇alkyl, for example n-pentyl. An aromatic radical R₁₀ has up to 20 carbon atoms and is unsubstituted or substituted, for example in each case unsubstituted or substituted naphthyl, such as especially 2-naphthyl, or preferably phenyl, the substituents preferably being selected from cyano, unsubstituted or hydroxy-, amino- or 4-methyl-piperazinyl-substituted lower alkyl, such as especially methyl, trifluoromethyl, free, etherified or esterified hydroxy, free, alkylated or acylated amino and free or esterified carboxy. In an aromatic-aliphatic radical R₁₀ the aromatic moiety is as defined above and the aliphatic moiety is preferably lower alkyl, such as especially C₁-C₂alkyl, which is substituted or preferably unsubstituted, for example benzyl. A cycloaliphatic radical R₁₀ has especially up to 30, more especially up to 20, and most especially up to 10 carbon atoms, is mono- or poly-cyclic and is substituted or preferably unsubstituted, for example such a cycloalkyl radical, especially such a 5- or 6-membered cycloalkyl radical, such as preferably cyclohexyl. In a cycloaliphatic-aliphatic radical R₁₀ the cycloaliphatic moiety is as defined above and the aliphatic moiety is preferably lower alkyl, such as especially C₁-C₂alkyl, which is substituted or preferably unsubstituted. A heterocyclic radical R₁₀ contains especially up to 20 carbon atoms and is preferably a saturated or unsaturated monocyclic radical having 5 or 6 ring members and 1-3 hetero atoms which are preferably selected from nitrogen, oxygen and sulfur, especially, for example, thienyl or 2-, 3- or 4-pyridyl, or a bi- or tri-cyclic radical wherein, for example, one or two benzene radicals are annellated (fused) to the mentioned monocyclic radical. In a heterocyclic-aliphatic radical R₁₀ the heterocyclic moiety is as defined above and the aliphatic moiety is preferably lower alkyl, such as especially C₁-C₂alkyl, which is substituted or preferably unsubstituted.

Etherified hydroxy is preferably lower alkoxy. Esterified hydroxy is preferably hydroxy esterified by an organic carboxylic acid, such as a lower alkanoic acid, or a mineral acid, such as a hydrohalic acid, for example lower alkanoyloxy or especially halogen, such as iodine, bromine or especially fluorine or chlorine.

Alkylated amino is, for example, lower alkylamino, such as methylamino, or di-lower alkylamino, such as dimethylamino. Acylated amino is, for example, lower alkanoylamino or benzoylamino.

Esterified carboxy is, for example, lower alkoxycarbonyl, such as methoxycarbonyl.

A substituted phenyl radical may carry up to 5 substituents, such as fluorine, but especially in the case of relatively large substituents is generally substituted by only from 1 to 3 substituents. Examples of substituted phenyl that may be given special mention are 4-chloro-phenyl, pentafluoro-phenyl, 2-carboxy-phenyl, 2-methoxy-phenyl, 4-fluoro-phenyl, 4-cyano-phenyl and 4-methyl-phenyl.

Salt-forming groups in a compound of formula I are groups or radicals having basic or acidic properties. Compounds having at least one basic group or at least one basic radical, for example a free amino group, a pyrazinyl radical or a pyridyl radical, may form acid addition salts, for example with inorganic acids, such as hydrochloric acid, sulfuric acid or a phosphoric acid, or with suitable organic carboxylic or sulfonic acids, for example aliphatic mono- or di-carboxylic acids, such as trifluoroacetic acid, acetic acid, propionic acid, glycolic acid, succinic acid, maleic acid, fumaric acid, hydroxymaleic acid, malic acid, tartaric acid, citric acid or oxalic acid, or amino acids such as arginine or lysine, aromatic carboxylic acids, such as benzoic acid, 2-phenoxy-benzoic acid, 2-acetoxy-benzoic acid, salicylic acid, 4-aminosalicylic acid, aromatic-aliphatic carboxylic acids, such as mandelic acid or cinnamic acid, heteroaromatic carboxylic acids, such as nicotinic acid or isonicotinic acid, aliphatic sulfonic acids, such as methane-, ethane- or 2-hydroxyethane-sulfonic acid, or aromatic sulfonic acids, for example benzene-, p-toluene- or naphthalene-2-sulfonic acid. When several basic groups are present mono- or poly-acid addition salts may be formed.

Compounds of formula I having acidic groups, for example a free carboxy group in the radical R₁₀, may form metal or ammonium salts, such as alkali metal or alkaline earth metal salts, for example sodium, potassium, magnesium or calcium salts, or ammonium salts with ammonia or suitable organic amines, such as tertiary monoamines, for example triethylamine or tri-(2-hydroxyethyl)-amine, or heterocyclic bases, for example N-ethyl-piperidine or N,N'-dimethyl-piperazine.

Compounds of formula I having both acidic and basic groups can form intemal salts.

Preference is given to COMPOUNDS OF THE INVENTION of formula I wherein one or two of the radicals R₄, R₅, R₆, R₇ and R₈ are each nitro or a radical of formula II
wherein
R₉ is hydrogen or lower alkyl,
X is oxo, thio, imino, N-lower alkyl-imino, hydroximino or O-lower alkyl-hydroximino,
Y is oxygen or the group NH,
n is 0 or 1 and
R₁₀ is an aliphatic radical having at least 5 carbon atoms or an aromatic, aromatic-aliphatic, cycloaliphatic, cycloaliphatic-aliphatic, heterocyclic or heterocyclic-aliphatic radical,
and the remaining radicals R₄, R₅, R₆, R₇ and R₈ are each independently of the others hydrogen, lower alkyl that is unsubstituted or substituted by free or alkylated amino, piperazinyl, piperidinyl, pyrrolidinyl or by morpholinyl, or lower alkanoyl, trifluoromethyl, free, etherified or esterifed hydroxy, free, alkylated or acylated amino or free or esterified carboxy,
and the remaining substituents are as defined above.

Preference is given especially to COMPOUNDS OF THE INVENTION of formula I
wherein
R₁ is pyridyl or N-oxido-pyridyl each of which is bonded at a carbon atom,
R₂ and R₃ are each hydrogen,
R₄ is hydrogen or lower alkyl,
R₅ is hydrogen, lower alkyl or trifluoromethyl,
R₆ is hydrogen,
R₇ is nitro, fluoro-substituted lower alkoxy or a radical of formula II wherein
R₉ is hydrogen,
X is oxo,
n is 0 and
R₁₀ is pyridyl bonded at a carbon atom, phenyl that is unsubstituted or substituted by halogen, cyano, lower alkoxy, carboxy, lower alkyl or by 4-methyl-piperazinyl-methyl, or C₅-C₇alkyl, thienyl, 2-naphthyl or cyclohexyl, and
R₈ is hydrogen.

Special preference is given to COMPOUNDS OF THE INVENTION of formula I wherein at least one of the radicals R₄ and R₈ is lower alkyl, and the remaining substituents are as defined above.

Preference is given above all to COMPOUNDS OF THE INVENTION of formula I wherein R₁ is pyridyl bonded at a carbon atom,
R₂, R₃, R₅, R₆ and R₈ are each hydrogen,
R₄ is lower alkyl,
R₇ a radical of formula II wherein
R₉ is hydrogen,
X is oxo,
n is 0 and
R₁₀ is 4-methyl-piperazinyl-methyl.

Preference is given above all especially to the COMPOUND I which is CGP 57148B {N-{5-[4-(4-methyl-piperazino-methyl)-benzoylamido]-2-methylphenyl)-4-(3-pyridyl)-2-pyrimidine-amine}. GP 57148B (hereinafter: "Imatinib" [International Non-proprietary Name]) and the use thereof, especially as an anti-tumour agent, are described in Example 21 of European patent application EP-A-0 564 409, which was published on 6 October 1993, and in equivalent applications and patents in numerous other countries, e.g. in US patent 5,521,184 and in Japanese patent 2706682. Another preference is given to the β-crystal form of 4-(4-methylpiperazin-1-ylmethyl)-N-[4-methyl-3-(4-pyridin-3-yl)pyrimidin-2-ylamino)phenyl]-benzamide methanesulfonate as described in the European patent application No. 998 473 published on May 10, 2000.

The term "4-(4-methylpiperazin-1-ylmethyl)-N-[4-methyl-3-(4-pyridin-3-yl)pyrimidin-2-yl-amino)phenyl]-benzamide" includes the β-crystal form as described in the European patent application No. 998 473.

Very preferably a N-phenyl-2-pyrimidine-amine derivatives of formula I is used in the form of its monomesylate salt.

The COMPOUNDS OF THE INVENTION of formula I are generically and specifically disclosed in the patent applications EP 0 564 409 A1 and WO 99/03854, in particular in the compound claims and the final products of the working examples, the subject-matter of the final products, the pharmaceutical preparations and the claims are hereby incorporated into the present application by reference to these publications. Comprised are likewise the corresponding stereoisomers as well as the corresponding polymorphs, e.g. crystal modifications, which are disclosed therein.

In EP 0 564 409 A1 the COMPOUNDS I are described to be useful for the therapy of cancer, thrombosis, psoriasis, fibrosis, dermatosclerosis and atherosclerosis. However, they have never been described as useful for the treatment of Ang II-mediated diseases such as cardiovascular hypertrophy or cardiovascular hypertrophic remodeling or hypertension-induced cardiovascular diseases. Artherosclerosis, fibrosis or restenosis and Ang II-mediated diseases such as cardiovascular hypertrophy, cardiovascular hypertrophic remodeling, hypertension-induced vascular diseases, or Ang II-mediated endothelial dysfunction with pro-inflammatory and pro-oxidant states are distinct diseases entities.

For the purposes of isolation or purification, as well as in the case of compounds that are used further as intermediates, it is also possible to use pharmaceutically unacceptable salts. Only pharmaceutically acceptable, non-toxic salts are used for therapeutic purposes, however, and those salts are therefore preferred.

Further suitable PDGF receptor tyrosine kinase inhibitors are disclosed in WO 98/35958, especially the compound of Example 62, and US 5,093,330 in each case in particular in the compound claims and the final products of the working examples, the subject-matter of which are hereby incorporated into the present application by reference to these publications. e.g. CGP57148B, CT52923 (4-(6,7-dimethoxy-4-quinazolinyl)-*N*-(3,4-methylenedioxybenzyl)-1-piperazinethiocarboxamide), RP-1776, GFB-111, pyrrolo[3,4-c]-beta-carboline-diones, SU 102 (developed by SUGEN), AG1296 (CAS Number 146535-11-7), AG1296 (CAS Number 71897-07-9), RPR101511A, etc.

CT52923 has been described byMatsuno K, et al. (1998) Synthesis and structure activity relationships of PDGF receptor phosphorylation inhibitor-1., in *18th Symposium on Medicinal Chemistry; 1998 Nov 25-27; Kyoto, Japan.* The Pharmaceutical Society of Japan, Division of Medicinal Chemistry, Tokyo, Japan. Abstract 2-P-05.

RP-1776, a novel cyclic peptide, was isolated from the culture broth of Streptomyces sp. KY11784. It was described by Toki S, Agatsuma T, et al, J Antibiot (Tokyo) 2001 May;54(5):405-14.

GFB-111 described by Blaskovich MA, et al.(Nat Biotechnol 2000 Oct;18(10):1065-70).

Pyrrolo[3,4-c]-beta-carboline-diones is described by Teller S, (Eur J Med Chem 2000 Apr;35(4):413-27).

The structure of the active agents identified by code nos., generic or trade names may be taken from the actual edition of the standard compendium "The Merck Index" or from databases, e.g. Patents International (e.g. IMS World Publications). The corresponding content thereof is hereby incorporated by reference.

Preferably, the present invention concerns the use of PDGF receptor tyrosine kinase inhibitors especially of N-phenyl-2-pyrimidine-amine derivatives of formula I or pharmaceutically acceptable salt thereof for the manufacture of a medicament for treating hypertension-induced and Ang II-mediated injuries, diseases involving angiotensin II induced hypertrophy or angiotensin II induced hypertrophic remodeling in the cardiovascular system and or in the kidney, angiotensin II induced renal damage and diseases, endothelial dysfunction with pro-inflammatory and pro-oxidant states.

Most preferably, the present invention concerns the use of PDGF receptor tyrosine kinase inhibitors especially of N-phenyl-2-pyrimidine-amine derivatives of formula I or pharmaceutically acceptable salt thereof for the manufacture of a medicament for treating diseases involving angiotensin II induced hypertrophy or angiotensin II induced hypertrophic remodeling in the cardiovascular system.

The present invention therefore concerns the use of PDGF receptor tyrosine kinase inhibitors especially of N-phenyl-2-pyrimidine-amine derivatives of formula I or pharmaceutically acceptable salt thereof for the manufacture of a medicament for treating congestive heart failure, heart failure, cardiac hypertrophy, cardiac remodeling after myocardial infarction, pulmonary congestion and cardiac fibrosis in dilated or in hypertrophic cardiomyopathy, hypertrophic cardiomyopathy, left or right ventricular hypertrophy, diabetic myopathy, stroke prevention in congestive heart failure, hypertrophic medial thickening in arteries and/or in large vessels, hypertension-induced vascular injuries, mesenteric vasculature hypertrophy.

Most preferably, the present invention concerns the use of PDGF receptor tyrosine kinase inhibitors especially of N-phenyl-2-pyrimidine-amine derivatives of formula I or pharmaceutically acceptable salt thereof for the manufacture of a medicament for treating renal hyperfiltration such as after portal renal ablation, proteinuria in chronic renal disease, renal arteriopathy as a consequence of hypertension, Nephrosclerosis or hypertensive nephrosclerosis, mesanglial hypertrophy.

In still another embodiment, the instant invention provides a method of treating a warm-blooded animal, especially a human, having or likely to contract a specified Ang II-mediated disease comprising administering to the animal a useful amount of COMPOUNDS OF THE INVENTION.

Preferably the COMPOUND OF THE INVENTION is imatinib; (4-(4-methylpiperazin-1-ylmethyl)-N-[4-methyl-3-(4-pyridin-3-yl)pyrimidin-2-ylamino)phenyl]-benzamide) or a pharmaceutically acceptable salt thereof especially in the form of its monomesylate salt.

The term "Ang II-mediated diseases" as used herein means but is not restricted to hypertension-induced and Ang II-mediated injury, diseases involving angiotensin II induced hypertrophy or angiotensin II induced hypertrophic remodeling in the cardiovascular system and or in the kidney, angiotensin II induced renal damage and diseases, endothelial dysfunction with pro-inflammatory and pro-oxidant states.

The term "angiotensin II induced renal damage and diseases" as used herein means but is not restricted to renal hyperfiltration such as after portal renal ablation, proteinuria in chronic renal disease, renal arteriopathy as a consequence of hypertension, Nephrosclerosis or hypertensive nephrosclerosis, mesanglial hypertrophy.

The term "endothelial dysfunction with pro-infilammatory and pro-oxidant states" as used herein means but is not restricted to chronic damage to the endothelium where Ang II is deeply involved.

The term "hypertension-induced and Ang II-mediated injury" as used herein means Ang II-mediated diseases in animals or humans suffering from hypertension such as hypertension-induced cardiovascular diseases, especially hypertension-induced vascular injuries.

The term "diseases involving angiotensin II induced hypertrophy or angiotensin II induced hypertrophic remodeling in the cardiovascular system or in the kidney" as used herein means but is not restricted to hypertrophic injuries especially congestive heart failure, heart failure, cardiac hypertrophy, cardiac remodeling after myocardial infarction, pulmonary congestion and cardiac fibrosis in dilated or in hypertrophic cardiomyopathy, hypertrophic cardiomyopathy, diabetic myopathy, stroke prevention in congestive heart failure, left or right ventricular hypertrophy, hypertrophic medial thickening in arteries and/or in large vessels, mesenteric vasculature hypertrophy.

Cardiac, vascular or kidney hypertrophy or hypertrophic remodeling is characterized by an increase in mass of the heart, arteries, large vessels or kidney.

Injuries in relation to hypertension caused by angiotensin II.
Hypertension, a condition of elevated blood pressure, affects a substantial number of the human population. Consequences of persistent hypertension include vascular damage to the ocular, renal, cardiac and cerebral systems, and the risk of these complications increases as blood pressure increases. Basic factors controlling blood pressure are cardiac output and peripheral vascular resistance, with the latter being the predominant common mechanism which is controlled by various influences. Injuries in relation to hypertension caused by angiotensin II, according to the invention are preferably but not limited to heart failure, heart failure, cardiac hypertrophy such as right or left ventricular hypertrophy (LVH) in hypertensive patients, renal arteriopathy, and vascular diseases.

Heart failure: The renin-angiotensin system (RAS) plays a pivotal role in the regulation of blood pressure and in the maintenance of fluid and electrolyte balance. Its individual components have been defined, and receptors stimulated by angiotensin II (Ang II) and their cellular responses have been characterized. The RAS is intimately involved in the pathogenesis and progression of HF. Most adverse effects of Ang II in HF are mediated via the Ang II type 1 receptor (AT₁R), a G-protein-coupled receptor containing 7-transmembrane regions. Left ventricular hypertrophy (LVH) is a nearly invariable accompaniment of heart failure related to hypertension.

Congestive heart failure (CHF; cardiac failure) is a condition in which weakened heart function exists together with a build-up of body fluid. Cardiac failure often occurs when cardiac output is insufficient to meet metabolic demands of the body, or when the heart cannot meet the demands of operating at increased levels of filling/diastolic pressure. Congestive heart failure may be caused by many forms of heart disease such as narrowing of the arteries supplying blood to the heart muscle (coronary heart disease); high blood pressure; primary disease of the heart muscle itself (cardiomyopathy); and infection of the heart valves and/or muscle itself (endocarditis and/or myocarditis). Each of these disease processes can lead to congestive heart failure by reducing the strength of the heart muscle contraction, by limiting the ability of the heart's pumping chambers to fill with blood due to mechanical problems or impaired diastolic relaxation, or by filling the heart's chambers with too much blood. Numerous compounds are known in the art to be useful for the prevention and treatment of congestive heart failure, including α- adrenergic antagonists, angiotensin II antagonists, angiotensin- converting enzyme (ACE) inhibitors, β-adrenergic antagonists, antihypertensives, calcium channel blockers, diuretics, potassium channel opening vasodilators, renin inhibitors, and serotonin antagonists.

Congestive heart failure may be associated with diabetes.

Cardiac hypertrophy or cardiac myocyte hypertrophy is an adaptive process of the heart to reduce wall stress when an increased pressure load is imposed on the myocardium. Activation of the renin-angiotensin system has been postulated to play a major role in the pathophysiology of cardiac hypertrophy and failure. In vivo studies have demonstrated that angiotensin II increases left ventricular mass and contributes to cardiac phenotype modulation independently from its effect on arterial pressure. By in vitro studies, it has been shown that angiotensin II causes cardiac myocyte hypertrophy and modulates interstitial fibrosis. Since in some animal models of experimental heart failure only an activation of the tissue renin-angiotensin system has been observed, whereas the circulating renin-angiotensin system has been unaltered, a special role has been suggested for the tissue renin-angiotensin system.

Adverse cardiac remodeling after myocardial infarction: The loss of contractile tissue by myocardial infarction (MI) is in part compensated for by hypertrophy of the remaining surviving myocardium. Larger transmural infarcts tend to expand and the whole ventricle reacts by dilatation. Adaptation to increased load of residual myocardium includes strengthening of the myocardium by increased collagen deposition. The whole process is termed remodeling of the heart (cardiac remodelling) and appears to be controlled by mechanical and humoral factors (angiotensin II levels). Importantly, remodeling includes gross morphologic, histologic, cellular, and molecular changes of the infarct and scar as well as of residual myocardium. Progressive dilatation is accompanied by progressive left ventricular (LV) dysfunction and electrical instability of the heart. Patients after acute MI are at risk of recurrent ischemia, arrhythmias, and remodeling even if they are asymptomatic. In the assessment of post-MI patients, it is important to identify the patient at risk of remodeling and to apply secondary preventive therapy that includes interruption or delay of the remodeling process.

Pulmonary congestion and cardiac fibrosis in dilated or in hypertrophic cardiomyopathy. Hypertrophic cardiomyopathy (HCM), the most common cause of sudden cardiac death in the young, is characterized by cardiac hypertrophy, myocyte disarray, and interstitial fibrosis.

Stroke prevention in congestive heart failure. The pathogenesis of hypertensive left ventricular hypertrophy (LVH) involves mechanical, hormonal, and neural factors. A number of earlier reports demonstrated that certain antihypertensive agents, although equipotent in lowering arterial pressure in hypertensive animals, may be more effective in reducing left ventricular (LV) mass than others and that the renin-angiotensin system may be involved independent of its pressor effect. This notion is further supported by the results of in vivo and in vitro studies showing that angiotensin II (Ang II) increases protein synthesis and myocardial mass and that this stimulatory effect of Ang II on myocardial growth may be blocked by specific Ang II receptor antagonists. In addition to these quantitative changes, LVH also involves the expression of cardiac genes and their respective proteins from the adult to the fetal pattern. Several proteins and their mRNAs are involved, including contractile proteins, creatine kinase, and atrial natriuretic peptide.

Nephrosclerosis or hypertensive nephrosclerosis is characterized by interstitial accumulation of the extracellular matrix components collagen types I, III, IV, V, and VI and fibronectin (remodeling). These matrix changes were accompanied by a progressive infiltration of mononuclear cells in the cortical interstitium, suggesting that the macrophages and T lymphocytes contribute to the development of fibrogenesis. The inhibition of the angiotensin II pathway is known to prevent Nephrosclerosis.

Renal arteriopathy as a consequence of hypertension. This disease leads to thickening of the arterial wall characterized by elastic disorganisation, hypertrophied smooth muscle cells and collagen bundles.

Proteinuria in chronic renal disease (independent of diabetes). lntraglomerular hypertension promotes proteinuria, which further activates the renin-angiotensin system (RAS). Angiotensin II, apart from its vasoconstrictor effects, induces local proinflammatory and profibrotic signaling molecules resulting in renal scarring.

Renal Hyperfiltration such as after portal renal ablation. Partial ablation of renal mass initiates a cycle of progressive glomerular injury in the remnant. This process is associated with glomerular hypertrophy, hyperfiltration and systemic hypertension.

In the present description, the term "treatment" includes both prophylactic or preventative treatment as well as curative or disease suppressive treatment, including treatment of patients at risk of contracting the disease or suspected to have contracted the disease as well as ill patients. This term further includes the treatment for the delay of progression of the disease.
The term "curative" as used herein means efficacy in treating ongoing diseases involving angiotensin II induced injuries.
The term "prophylactic" means the prevention of the onset or recurrence of diseases involving angiotensin II induced injuries.
The term "delay of progression" as used herein means administration of the active compound to patients being in a pre-stage or in an early phase of the disease to be treated, in which patients for example a pre-form of the corresponding disease is diagnosed or which patients are in a condition, e.g. during a medical treatment or a condition resulting from an accident, under which it is likely that a corresponding disease will develop.

Possible pharmaceutical preparations, containing an effective amount of COMPOUND I are described in PCT patent application WO99/03854 published on January 28, 1999.
Depending on species, age, individual condition, mode of administration, and the clinical picture in question, effective doses, for example daily doses of about 100-1000 mg, preferably 200-600 mg, especially 400 mg, are administered to warm-blooded animals of about 70 kg bodyweight. For adult patients a starting dose of 400 mg daily can be recommended. For patients with an inadequate response after an assessment of response to therapy with 400 mg daily, dose escalation can be safely considered and patients may be treated as long as they benefit from treatment and in the absence of limiting toxicities. Two pharmaceutical compositions are described in examples 2 and 3.

The invention relates also to a method for administering to a human, subject to angiotensin II-mediated diseases, Imatinib or a pharmaceutically acceptable salt thereof, which comprises administering a pharmaceutically effective amount of Imatinib or a pharmaceutically acceptable salt thereof to the human subject once daily for a specific period. The invention relates especially to such method wherein a daily dose of 200 to 600 mg, especially 400-600 mg, preferably 400 mg, of Imatinib mesylate is administered.

Surprisingly, it has been found that the delay of progression or treatment of hypertension-induced diseases, in particular the treatment cardiovascular damages (i.e. hypertension induced cardiovascular hypertrophy or cardiovascular hypertrophic remodeling), of a combination as defined herein is greater than the effects that can be achieved with either type of combination partner alone, i.e. greater than the effects of a monotherapy using only one of the combination partners (a) and (b) as defined herein.

The applicant has surprisingly discovered that the combination of COMPOUNDS OF THE INVENTION especially Compound I and at least one compound selected from an antihypertensive, an aldosterone antagonist, an aldosterone synthase inhibitor and/or an angiotensin receptor blocker agent has an unexpected high benefic effect on the prevention or the treatment of hypertension-induced diseases.

Thus this invention concerns a combination, such as a combined preparation or a pharmaceutical composition, which comprises (a) at least one COMPOUND OF THE INVENTION preferably N-{5-[4-(4-methyl-piperazino-methyl)-benzoylamido]-2-methylphenyl}-4-(3-pyridyl)-2-pyrimidine-amine, and at least one compound selected from (b) an antihypertensive, an aldosterone antagonist, an aldosterone synthase inhibitor and/or an angiotensin receptor blocker agent in which the active ingredients are present independently of each other in free form or in the form of a pharmaceutically acceptable salt and optionally at least one pharmaceutically acceptable carrier; for simultaneous, separate or sequential use. Such a combination will be referred hereinafter as COMBINATION OF THE INVENTION.

Thus in still another embodiment, the instant invention provides a method of treating a warm-blooded animal, especially a human, having or likely to contract a hypertensive-mediated disease in particular the treatment of cardiovascular damages (i.e hypertension induced cardiovascular hypertrophy or cardiovascular hypertrophic remodeling), comprising administering to the animal a combination, such as a combined preparation or a pharmaceutical composition, which comprises (a) at least one COMPOUND OF THE INVENTION preferably N-{5-[4-(4-methyl-piperazino-methyl)-benzoylamido]-2-methylphenyl}-4-(3-pyridyl)-2-pyrimidine-amine, and at least one compound selected from (b) an antihypertensive, an aldosterone antagonist, an aldosterone synthase inhibitor and/or an angiotensin receptor blocker agent in which the active ingredients are present independently of each other in free form or in the form of a pharmaceutically acceptable salt and optionally at least one pharmaceutically acceptable carrier.

Preferably the active ingredients are present in a quantity, which is jointly therapeutically effective against hypertensive-mediated disease in particular the treatment of cardiovascular damages.

In a further preferred aspect, this inventions concerns a combination, use or method as described above, wherein (a) is at least one COMPOUND OF THE INVENTION and (b) is Valsartan and optionally Hydrochlorothiazide. Surprisingly, Valsartan as combination partner (b) exhibits a beneficial and unexpected effect, e.g., a mutual enhancing of the effect of the combination partners (a) and (b), in particular a synergism, e.g. a more than additive effect, additional advantageous effects, less side effects, a combined therapeutical effect in a non-effective dosage of one or both of the combination partners (a) and (b), and very preferably a strong synergism of the combination partners (a) and (b).

The term "a combined preparation", as used herein defines especially a "kit of parts" in the sense that the combination partners (a) and (b) as defined above can be dosed independently or by use of different fixed combinations with distinguished amounts of the combination partners (a) and (b), i.e., simultaneously or at different time points. The parts of the kit of parts can then, e.g., be administered simultaneously or chronologically staggered, that is at different time points and with equal or different time intervals for any part of the kit of parts. Very preferably, the time intervals are chosen such that the effect on the treated disease in the combined use of the parts is larger than the effect which would be obtained by use of only any one of the combination partners (a) and (b). The ratio of the total amounts of the combination partner (a) to the combination partner (b) to be administered in the combined preparation can be varied, e.g. in order to cope with the needs of a patient sub-population to be treated or the needs of the single patient which different needs can be due to the particular disease, age, sex, body weight, etc. of the patients. Preferably, there is at least one beneficial effect, e.g., a mutual enhancing of the effect of the combination partners (a) and (b), in particular a synergism, e.g. a more than additive effect, additional advantageous effects, less side effects, a combined therapeutical effect in a non-effective dosage of one or both of the combination partners (a) and (b), and very preferably a strong synergism of the combination partners (a) and (b).

The combinations of the present invention inhibit hypertensive-mediated disease in particular the treatment of cardiovascular damages (i.e hypertension induced cardiovascular hypertrophy or cardiovascular hypertrophic remodeling). Furthermore, depending the particular combination used different decreases of the cardiovascular hypertrophy or cardiovascular hypertrophic remodeling can be obtained. The combinations disclosed herein are also suited to prevent endothelial dysfunction with pro-inflammatory and pro-oxidant states and hypertension-induced vascular injuries. The combinations disclosed herein are in particular suitable for the treatment of hypertension-induced vascular injuries such as vascular hypertrophy or hypertrophic remodeling.

The term "Hypertension-mediated diseases" as used herein means but is not restricted to diseases involving angiotensin II induced hypertrophy or angiotensin II induced hypertrophic remodeling in the cardiovascular system and or in the kidney, angiotensin II induced renal damage and diseases, endothelial dysfunction with pro-inflammatory and pro-oxidant states, hypertension-induced vascular injuries, hypertension-induced cardiovascular injuries.

The term "hypertension induced cardiovascular hypertrophy or cardiovascular hypertrophic remodeling "as used herein mean but are not restricted to hypertrophic injuries especially congestive heart failure, heart failure, cardiac hypertrophy, cardiac remodeling after myocardial infarction, pulmonary congestion and cardiac fibrosis in dilated or in hypertrophic cardiomyopathy, stroke prevention in congestive heart failure, hypertrophic medial thickening in arteries and/or in large vessels, mesenteric vasculature hypertrophy.

It will be understood that references to the combination partners (a) and (b) are meant to also include the pharmaceutically acceptable salts. If these combination partners (a) and (b) have, for example, at least one basic center, they can form acid addition salts. Corresponding acid addition salts can also be formed having, if desired, an additionally present basic center. The combination partners (a) and (b) having an acid group (for example COOH) can also form salts with bases. The combination partner (a) or (b) or a pharmaceutically acceptable salt thereof may also be used in form of a hydrate or include other solvents used for crystallization. N-{5-[4-(4-methyl-piperazino-methyl)-benzoylamido]-2-methylphenyl}-4-(3-pyridyl)-2-pyrimidine-amine, i.e. combination partner (a), is preferably used in the present invention in the form of its monomesylate salt (STI571).

The combination partner (a) or COMPOUND I can be prepared and administered as described in WO 99/03854, especially the monomesylate salt of N-{5-[4-(4-methyl-piperazino-methyl)-benzoylamido]-2-methylphenyl}-4-(3-pyridyl)-2-pyrimidine-amine can be formulated as described in Examples 4 and 6 of WO 99/03854.

The term "COMPOUND I" includes all the pharmaceutically acceptable salt thereof and may also be used in form of a hydrate or includes crystal forms such as described in the European patent application No. 998 473 published on May 10, 2000.

The structure of the active agents identified by code nos., generic or trade names may be taken from the actual edition of the standard compendium "The Merck Index" or from databases, e.g. Patents international (e.g. IMS World Publications).

A combination which comprises (a) at least one COMPOUND OF THE INVENTION preferably N-{5-[4-(4-methyl-piperazino-methyl)-benzoylamido]-2-methylphenyl}-4-(3-pyridyl)-2-pyrimidine-amine and at least one compound selected from (b) an antihypertensive, an aldosterone antagonist, an aldosterone synthase inhibitor and/or an angiotensin receptor blocker agent, in which the active ingredients are present in each case in free form or in the form of a pharmaceutically acceptable salt and optionally at least one pharmaceutically acceptable carrier, will be referred to hereinafter as a COMBINATION OF THE INVENTION.

All the more surprising is the experimental finding that *in vivo* the administration of a COMBINATION OF THE INVENTION, especially comprising an angiotensin receptor blocker as combination partner (b), results not only in a beneficial effect, especially a synergistic therapeutic effect, e.g. with regard to slowing down, arresting or reversing the hypertension-mediated disease especially the hypertrophic and vascular injuries, but also in further surprising beneficial effects, e.g. less side-effects, an improved quality of life and a decreased mortality and morbidity, compared to a monotherapy applying only one of the pharmaceutically active ingredients used in the COMBINATION OF THE INVENTION, in particular in the treatment of hypertensive-mediated disease most particularly the treatment of cardiovascular damages (i.e hypertension induced cardiovascular hypertrophy or cardiovascular hypertrophic remodeling). In particular, an increased up-take of the combination partner (b) in cells is observed, when applied in combination with combination partner (a).

A further benefit is that lower doses of the active ingredients of the COMBINATION OF THE INVENTION can be used, for example, that the dosages need not only often be smaller but are also applied less frequently, or can be used in order to diminish the incidence of side-effects. This is in accordance with the desires and requirements of the patients to be treated.

It can be shown by established test models and in particular those test models described herein that a COMBINATION OF THE INVENTION results in a more effective delay of progression or treatment of hypertensive-mediated disease in particular the treatment of cardiovascular damages (i.e hypertension induced cardiovascular hypertrophy or cardiovascular hypertrophic remodeling), compared to the effects observed with the single combination partners. The person skilled in the pertinent art is fully enabled to select a relevant test model to prove the hereinbefore and hereinafter mentioned therapeutic indications and beneficial effects. The pharmacological activity of a COMBINATION OF THE INVENTION may, for example, be demonstrated in a clinical study or in a test procedure as essentially described hereinafter.

Suitable clinical studies are, for example, open label non-randomized, dose escalation studies in patients with advanced hypertension. Such studies prove in particular the synergism of the active ingredients of the COMBINATIONS OF THE INVENTION. The beneficial effects on Hypertension-mediated diseases can be determined directly through the results of these studies or by changes in the study design which are known as such to a person skilled in the art. Such studies are, in particular, suitable to compare the effects of a monotherapy using the active ingredients and a COMBINATION OF THE INVENTION. Preferably, the combination partner (a) is administered with a fixed dose and the dose of the combination partner (b) is escalated until the Maximum Tolerated Dosage is reached. In a preferred embodiment of the study, each patient receives daily doses of the combination partner (a). The efficacy of the treatment can be determined in such studies, e.g., after 18 or 24 weeks by evaluation of the cardiovascular system every 6 weeks.

Alternatively, a placebo-controlled, double blind study can be used in order to prove the benefits of the COMBINATION OF THE INVENTION mentioned herein.

The antihypertensive agent is selected from but not limited to a diuretic, an angiotensin converting enzyme inhibitor, a calcium channel blocking drug, HMG-Co-A reductase
inhibitor, dual angiotensin converting enzyme/neutral endopetidase (ACE/NEP) inhibitor, endothelin antagonist, vasodilators and an β-adrenergic blocking drug.

A diuretic is, for example, a thiazide derivative selected from the group consisting of chlorothiazide, hydrochlorothiazide, furosemide, diuril, amiloride, hydrodiuril , methylclothiazide, and chlorothalidon. The most preferred is hydrochlorothiazide.

The interruption of the enzymatic degradation of angiotensin I to angiotensin II with so-called ACE-inhibitors (also called angiotensin converting enzyme inhibitors) is a successful variant for the regulation of blood pressure and thus also makes available a therapeutic method for the treatment of congestive heart failure. The class of ACE inhibitors comprises compounds having differing structural features. For example, mention may be made of the compounds which are selected from the group consisting alacepril, benazepril, benazeprilat, captopril, ceronapril, cilazapril, delapril, enalapril, enaprilat, fosinopril, imidapril, lisinopril, moveltopril, perindopril, quinapril, ramipril, spirapril, temocapril, and trandolapril, or, in each case, a pharmaceutically acceptable salt thereof. Preferred ACE inhibitors are those agents that have been marketed, most preferred are benazepril and enalapril.

The class of CCBs (calcium channel blockers) essentially comprises dihydropyridines (DHPs) and non-DHPs such as diltiazem-type and verapamil-type CCBs.
A CCB useful in said combination is preferably a DHP representative selected from the group consisting of amlodipine, felodipine, ryosidine, isradipine, lacidipine, nicardipine, nifedipine, niguldipine, niludipine, nimodipine, nisoldipine, nitrendipine, and nivaldipine, and is preferably a non-DHP representative selected from the group consisting of flunarizine, prenylamine, diltiazem, fendiline, gallopamil, mibefradil, anipamil, tiapamil and verapamil, and in each case, a pharmaceutically acceptable salt thereof. All these CCBs are therapeutically used, e.g. as anti-hypertensive, anti-angina pectoris or anti-arrhythmic drugs. Preferred CCBs comprise amlodipine, diltiazem, isradipine, nicardipine, nifedipine, nimodipine, nisoldipine, nitrendipine, and verapamil, or, e.g. dependent on the specific CCB, a pharmaceutically acceptable salt thereof. Especially preferred as DHP is amlodipine or a pharmaceutically acceptable salt, especially the besylate, thereof. An especially preferred representative of non-DHPs is verapamil or a pharmaceutically acceptable salt, especially the hydrochloride, thereof.

HMG-Co-A reductase inhibitors (also called β-hydroxy-β-methylglutaryl-co-enzyme-A reductase inhibitors) are understood to be those active agents that may be used to lower the lipid levels including cholesterol in blood. The class of HMG-Co-A reductase inhibitors comprises compounds having differing structural features. For example, mention may be made of the compounds that are selected from the group consisting of atorvastatin, cerivastatin, compactin, dalvastatin, dihydrocompactin, fluindostatin, fluvastatin, lovastatin, pitavastatin, mevastatin, pravastatin, rivastatin, simvastatin, and velostatin, or, in each case, a pharmaceutically acceptable salt thereof. Preferred HMG-Co-A reductase inhibitors are those agents which have been marketed, most preferred is fluvastatin and pitavastatin and also atorvastatin or, in each case, a pharmaceutically acceptable salt thereof.

Compounds having an inhibitory effects on both angiotensin converting enzyme and neutral endopetidase, so-called dual ACE/NEP inhibitors, can be used for the treatment of cardiovascular pathologies. A preferred dual angiotensin converting enzyme/neutral endopetidase (ACE/NEP) inhibitor is, for example, omapatrilate (cf. EP 629627), fasidotril or fasidotrilate, or Z 13752A (cf. WO 97/24342) or, if appropriable, a pharmaceutically acceptable salt thereof.

Endothelin (ET) is a highly potent vasoconstrictor peptided synthesized and released by the vascular endotleium. Endothelin exists in three isoforms (ET-1, ET-2 and ET-3). (ET shall meand any or all othe isoforms of ET). Elevated levels of ET have been reported in plasma fomr patients with e.g. essential hypertension. Endothelin receptor antagonist can be used to inhibit the vasoconstrictive effects induced by ET. A preferred endothelin antagonist is, for example, bosentan (cf. EP 526708 A), enrasentan (cf. WO 94/25013), atrasentan (cf. WO 96/06095), especially atrasentan hydrochloride, darusentan (cf. EP 785926 A), BMS 193884 (cf. EP 702012 A), sitaxentan (cf. US 5594021), especially sitaxsentan sodium, YM 598 (cf. EP 882719 A), S 0139 (cf. WO 97/27314), J 104132 (cf. EP 714897 A or WO 97/37665), furthermore, tezosentan (cf. WO 96/19459), or in each case, a pharmaceutically acceptable salt thereof.

Examples of known β-adrenergic receptor antagonists include atenolol, propranolol, timolol, and metoprolol.

AT₁-receptor antagonists (also called angiotensin II receptor antagonists) are understood to be those active ingredients that bind to the AT₁-receptor subtype of angiotensin II receptor but do not result in activation of the receptor. As a consequence of the inhibition of the AT₁ receptor, these antagonists can, for example, be employed as antihypertensives or for treating congestive heart failure.

The class of AT₁ receptor antagonists comprises compounds having differing structural features, essentially preferred are the non-peptidic ones. For example, mention may be made of the compounds that are selected from the group consisting of valsartan (as described in the European patent application No. 0 443 983 or the US patent No. 5,399,578 - CAS : 137862-53-4, trade name: Diovan), losartan (described in the European patent application No. EP25331 0), candesartan (described in the European patent application No. 459136), eprosartan (described in the European patent application No. 403159), irbesartan (described in the European patent application No. 454511), olmesartan (described in the European patent application No. 503785), tasosartan (described in the European patent application No. 539086), telmisartan (described in the European patent application No. 522314), cilexetil , the compound with the designation E-1477, SC-52458, ZD-8731 or a pharmaceutically acceptable salt thereof.

In a preferred embodiment of the invention the Aldosterone antagonist is selected from but not limited to spironolactones and a group of potassium sparing diuretics that acts by inhibiting aldosterone.

Aldosterone synthase inhibitor is an enzyme that converts corticosterone to aldosterone by hydroxylating cortocosterone to form 18-OH-corticosterone and 18-OH-corticosterone to aldosterone. The class of aldosterone synthase inhibitors is known to be applied for the treatment of hypertension and primary aldosteronism comprises both steroidal and non-steroidal aldosterone synthase inhibitors, the later being most preferred.

Preference is given to commercially available aldosterone synthase inhibitors or those aldosterone synthase inhibitors that have been approved by the health authorities.

The class of aldosterone synthase inhibitors comprises compounds having differing structural features. For example, mention may be made of the compounds which are selected from the group consisting of the non-steroidal aromatase inhibitors anastrozole, fadrozole (including the (+)-enantiomer thereof), as well as the steroidal aromatase inhibitor exemestane, or, in each case where applicable, a pharmaceutically acceptable salt thereof.

The most preferred non-steroidal aldosterone synthase inhibitor is the (+)-enantiomer of the hydrochloride of fadrozole (US patents No. 4617307 and 4889861), A preferred steroidal aldosterone antagonist is eplerenone (cf. EP 122232 A).

Preferably, the COMBINATION OF THE INVENTION comprises as combination partner (b) at least one compound selected from the group consisting of valsartan, fluvastatin, atorvastatin, pitavastatin, benzepril, enalapril, amlodipine, especially the besylate thereof, the (+) enantiomer of fadrozole, eplerenone, omapatrilate, Z 13752A, sitaxsentan, especially sitaxsentan sodium, darusentan and hydrochlorothiazide.

In a best embodiment, the angiotensin receptor blocker is Valsartan. Valsartan is a potent, orally active angiotensin II receptor antagonist and which at doses of 80 and 160 mg once daily has been shown to be as effective and better tolerated as commonly used ACE inhibitors, including enalapril, for the treatment of mild to moderate essential hypertension. The preferred daily dosage according to the COMBINATION OF THE INVENTION is between 40 and 180 mg, preferably 80 to 160 mg.

In a preferred embodiment, the number of combination partner (b) is between 1 and 3, most preferably the COMBINATION OF THE INVENTION comprises one or two combination partner (b).

Furthermore, the present invention pertains to the use of a COMBINATION OF THE INVENTION for the prevention, the delay of progression or treatment of hypertension-induced diseases, in particular the treatment cardiovascular damages (i.e hypertension induced cardiovascular hypertrophy or cardiovascular hypertrophic remodeling) and for the preparation of a medicament for the delay of progression or treatment of hypertension-induced diseases, in particular the treatment cardiovascular damages (i.e hypertension induced cardiovascular hypertrophy or cardiovascular hypertrophic remodeling).

Most preferably, in such embodiment the COMBINATION OF THE INVENTION comprises an angiotensin receptor blocker, especially Valsartan (CAS : 137862-53-4).

Another embodiment of the invention relates to the use of a COMBINATION OF THE INVENTION for the preparation of a medicament for the prevention, delay of progression or treatment of or for the preparation of a medicament for the prevention, delay of progression or treatment of endothelial dysfunction with pro-inflammatory and pro-oxidant states.

The COMBINATION OF THE INVENTION can be a combined preparation or a pharmaceutical composition.

It is one objective of this invention to provide a pharmaceutical composition comprising a quantity, which is jointly therapeutically effective against hypertension-induced diseases, in particular the treatment cardiovascular damages (i.e hypertension induced cardiovascular hypertrophy or cardiovascular hypertrophic remodeling) comprising the COMBINATION OF THE INVENTION. In this composition, the combination partners (a) and (b) can be administered together, one after the other or separately in one combined unit dosage form or in two separate unit dosage forms. The unit dosage form may also be a fixed combination.

The pharmaceutical compositions for separate administration of the combination partners (a) and (b) and for the administration in a fixed combination, i.e. a single galenical compositions comprising at least two combination partners (a) and (b), according to the invention can be prepared in a manner known per se and are those suitable for enteral, such as oral or rectal, and parenteral administration to mammals (warm-blooded animals), including man, comprising a therapeutically effective amount of at least one pharmacologically active combination partner alone or in combination with one or more pharmaceutically acceptable carries, especially suitable for enteral or parenteral application.

Novel pharmaceutical composition contain, for example, from about 10 % to about 100 %, preferably from about 20 % to about 60 %, of the active ingredients. Pharmaceutical preparations for the combination therapy for enteral or parenteral administration are, for example, those in unit dosage forms, such as sugar-coated tablets, tablets, capsules or suppositories, and furthermore ampoules. If not indicated otherwise, these are prepared in a manner known per se, for example by means of conventional mixing, granulating, sugar-coating, dissolving or lyophilizing processes. It will be appreciated that the unit content of a combination partner contained in an individual dose of each dosage form need not in itself constitute an effective amount since the necessary effective amount can be reached by administration of a plurality of dosage units.

In particular, a therapeutically effective amount of each of the combination partner of the COMBINATION OF THE INVENTION may be administered simultaneously or sequentially and in any order, and the components may be administered separately or as a fixed combination. For example, the method of delay of progression or treatment of hypertension-induced diseases, in particular the treatment cardiovascular damages (i.e hypertension induced cardiovascular hypertrophy or cardiovascular hypertrophic remodeling) according to the invention may comprise (i) administration of the combination partner (a) in free or pharmaceutically acceptable salt form and (ii) adminstration of a combination partner (b) in free or pharmaceutically acceptable salt form, simultaneously or sequentially in any order, in jointly therapeutically effective amounts, preferably in synergistically effective amounts, e.g. in daily dosages corresponding to the amounts described herein. The individual combination partners of the COMBINATION OF THE INVENTION can be administered separately at different times during the course of therapy or concurrently in divided or single combination forms. Furthermore, the term administering also encompasses the use of a pro-drug of a combination partner that convert *in vivo* to the combination partner as such. The instant invention is therefore to be understood as embracing all such regimes of simultaneous or alternating treatment and the term "administering" is to be interpreted accordingly.

The effective dosage of each of the combination partners employed in the COMBINATION OF THE INVENTION may vary depending on the particular compound or pharmaceutical composition employed, the mode of administration, the condition being treated, the severity of the condition being treated. Thus, the dosage regimen the COMBINATION OF THE INVENTION is selected in accordance with a variety of factors including the route of administration and the renal and hepatic function of the patient. A physician, clinician or veterinarian of ordinary skill can readily determine and prescribe the effective amount of the single active ingredients required to prevent, counter or arrest the progress of the condition. Optimal precision in achieving concentration of the active ingredients within the range that yields efficacy without toxicity requires a regimen based on the kinetics of the active ingredients' availability to target sites.

N-{5-[4-(4-methyl-piperazino-methyl)-benzoylamido]-2-methylphenyl}-4-(3-pyridyl)-2-pyrimidine-amine monomesylate, is preferably administered to a human in a dosage in the range of about 2.5 to 850 mg/day, more preferably 5 to 600 mg/day and most preferably 20 to 300 mg/day. Unless stated otherwise herein, the compound is preferably administered from one to four times per day.

Valsartan may be administered orally to a human in a dosage range varying from about 40 to about 160 mg/day, preferably from about 60 to about 160 mg/day.

Hydrochlorothiazide may be administered orally to a human in a dosage range varying from about 4 to about 40 mg/day, preferably from about 8 to about 20 mg/day.

Moreover, the present invention provides a commercial package comprising as active ingredients COMBINATION OF THE INVENTION, together with instructions for simultaneous, separate or sequential use thereof in the delay of progression or treatment of hypertension-induced diseases, in particular the treatment cardiovascular damages (i.e hypertension induced cardiovascular hypertrophy or cardiovascular hypertrophic remodeling).

The term "a commercial package", as used herein defines especially a "kit of parts" in the sense that the components (a) and (b) as defined above can be dosed independently or by use of different fixed combinations with distinguished amounts of the components (a) and (b), i.e., simultaneously or at different time points. Moreover, these terms comprise a commercial package comprising (especially combining) as active ingredients components (a) and (b), together with instructions for simultaneous, sequential (chronically staggered, in time-specific sequence, preferentially) or (less preferably) separate use thereof in the delay of progression or treatment of hypertension-mediated diseases. The parts of the kit of parts can then, e.g., be administered simultaneously or chronologically staggered, that is at different time points and with equal or different time intervals for any part of the kit of parts. Very preferably, the time intervals are chosen such that the effect on the treated disease in the combined use of the parts is larger than the effect which would be obtained by use of only any one of the combination partners (a) and (b) (as can be determined according to standard methods, e.g. the determination of Combination Index or the use of isobolograms). The ratio of the total amounts of the combination partner (a) to the combination partner (b) to be administered in the combined preparation can be varied, e.g. in order to cope with the needs of a patient sub-population to be treated or the needs of the single patient which different needs can be due to the particular disease, age, sex, body weight, etc. of the patients. Preferably, there is at least one beneficial effect, e.g., a mutual enhancing of the effect of the combination partners (a) and (b), in particular a more than additive effect, which hence could be achieved with lower doses of each of the combined drugs, respectively, than tolerable in the case of treatment with the individual drugs only without combination, producing additional advantageous effects, e.g. less side effects or a combined therapeutical effect in a non-effective dosage of one or both of the combination partners (components) (a) and (b), and very preferably a strong synergism (Combination Index above 4) of the combination partners (a) and (b).

The following Example illustrates the invention described above, but is not, however, intended to limit the scope of the invention in any way. The beneficial effects of the COMBINATION OF THE INVENTION can also be determined by other test models known as such to the person skilled in the pertinent art or by clinical trials.

The person skilled in the pertinent art is fully enabled to select a relevant test model to prove the herein indicated therapeutic indications and beneficial effects (i.e. good therapeutic margin, no action on hypertension, and other advantages). The pharmacological activity may, for example, be demonstrated in a clinical study or in the test procedure as essentially described hereinafter in a manner known to the skilled person.

### EXPERIMENTAL PART

### Example 1:

Animals: Forty-eight, 8 week old, male Sprague Dawley rats were anaesthetized with enflurane (Abbott Australasia, Kumal, NSW, Australia) and a osmotic minipump (Alzet Model 2002, Alzet Corporation, Palo Alto) inserted in the interscapular region. Rats were randomized to receive minipumps filled vehicle (0.15 M NaCl, 1 mmol/l acetic acid) either with or without angiotensin II (58 ng/min), as previously described (Cao Z et col. *Kidney Int* 58:2437-2451, 2000). Animals were then further randomized to receive either no treatment or COMPOUND I at a dose of 60 mg/kg by daily gavage and sacrificed 12 days later. Rats had unrestricted access to water and standard rat chow. Systolic blood pressure (SBP) was measured by indirect tail-cuff plethysmography in prewarmed unanesthetized animals as previously described (Bunag RD; *Journal of Applied Physiology* 34:279-282, 1973). In each group of 16 animals, 8 were used for histological studies and eight were used for gene expression analyses. In the latter group, animals were sacrificed by decapitation after which the mesenteric vessels were removed and stripped of surrounding fat, connective tissue and veins to yield the superior mesenteric arterial tree as previously described (Rumble J et al.; J *Hypertension* 14:601-607, 1996). The vessels were weighed, snap frozen in liquid nitrogen and subsequently stored at -80°C.

Histochemistry and immunohistochemistry: Histological studies of vascular architecture were performed in a subset of animals (n = 8/group). Animals in this subgroup were anesthetized with pentobarbital sodium (Nembutal, Bomac Laboratories, Asquith, Australia) and vessels were perfused at arterial pressure with 10% neutral buffered formalin via an intra-aortic cannula. Tissues were then prepared as previously described (Cooper ME et col.; *Diabetes* 43:1221-1228, 1994). In brief, mesenteric vessels were placed in ice cold phosphate buffer where fat, connective tissue and veins were removed by blunt dissection. The resultant vessel preparation was then embedded in paraffin. Sections were then either histochemically or immunohistochemically stained. Histochemical staining comprised the use of either haematoxlyin and eosin or Masson's trichrome (Masson P; *J Tech Methods* 2:75-90, 1929) to examine extracellular matrix. Smooth muscle was immunostained using a polyclonal α-smooth muscle actin antibody (Biogenes, San Ramon, CA) and immunohistochemistry was performed using the indirect avidin-biotin complex method as previously described (Rumble JR et cal.; *J Clin Invest* 99:1016-1027, 1997).

Quantification of histopathology: The proportion of vessel wall occupied by the media was identified by immunolabelling for α-smooth muscle actinand the ratio of the wall:lumen quantified using of a video-imaging system (Video Pro 32, Leading Edge, Bedford Park, South Australia, Australia), connected to a light microscope (Zeiss, Oberkocken, Germany) with photographic attachment (Axiophot, Zeiss, Oberkocken, Germany). Using this device, the media (areas stained with the anti-actin antibody) and corresponding lumenal areas were determined in a median of 20 (range 10-35) vessels per animal and expressed as wall:lumen ratio, as previously described in other models of vascular hypertrophy (Kakinuma Yet col.; *Kidney Int* 42:46-55., 1992).

Quantification of ECM was performed on trichrome-stained sections by calculating the proportion of area occupied by ECM using computer-assisted image analysis as previously described (Lehr HA, et col. *J Histochem Cytochem* 45:1559-1565, 1997). In brief, the color ranges for matrix (blue on trichrome stained sections), media smooth muscle (red on trichrome stained sections) were selected and image analysis was performed using a chromogen-separating technique (Lehr HA, et col.). The entire section was examined by light microscopy (Olympus BX-50, Olympus Optical, Tokyo, Japan) and digitized using a high resolution camera (Fujix HC-2000, Fujifilm, Tokyo, Japan). All images were obtained using a 20X objective lens. Digitized images were then captured on a Power Macintosh G3 computer (Apple Computer Inc., Cupertino, CA) equipped with an in-built graphic board and evaluated using analytical software (Analytical Imaging Software, Ontario).

RNA Extraction and cDNA synthesis :Frozen mesenteric vascular tissue, stored at -80°C was homogenized (Ultra-Turrax, Janke and Kunkel, Staufen, Germany) and total RNA was isolated by the acid guanidinium thiocyanate-phenol-chloroform extraction method (Chomczynski P, Sacchi N; *Anal Biochem* 162:156-159, 1987). The purified RNA was dissolved in sterile water, quantified spectrophotometrically and its concentration adjusted to 1 µg/µl. Ten micrograms of total RNA in 10µl of water was mixed with 1 µl (0.05µg) random hexamers (Life Technologies, Rockville, MD) and incubated for 10 mins at 70°C. After cooling on ice for 2 minutes, 2µl of 10 × PCR buffer, 2µl of 25mM of MgCl₂, 1µl of 10mM dNTP mix and 2µl of 0.1 M DTT were added in order. Tubes were incubated at 25°C for 5 min after which 1µl of SuperScript II reverse transcriptase (200U; Life Technologies, Rockville, MD) and incubated at 25°C for 10 min and at 42°C for 50 min. Reactions were terminated at 70°C for 15 min before chilling on ice. One microliter of RNase H was added to each sample at incubated at 37°C after which samples were stored at -40°C for future use. Parallel reactions for each RNA sample were run in the absence of SuperScript II (genomic controls) to assess the degree of any contaminating genomic DNA.

Quantitative Real Time RT-PCR: Transforming growth factor-β1 (TGF-β1) and platelet-derived growth factor (PDGF) gene expression was quantified by real-time RT-PCR using sequence specific primers as previously reported by our group (Ahola H, et col.; *American journal of Pathology* 155:907-913, 1999). A commercial, pre-developed 18S control kit labeled with the fluorescent reporter dye [VIC] on the 5' end and the quencher [TAMRA] on the 3' end (PE Biosystems, Foster City, CA) was used as the housekeeping gene to control for inequalities of loading. Primers and probes for target genes were obtained from PE Biosystems (Foster City, CA). Both probes included a fluorescence reporter (6-carboxyfluorescein [FAM]) at the 5'-end and a fluorescent quencher (6-carboxytetramethylrhodamine [TAMRA]) at the 3'-end. For the relative quantification of the target gene and the endogenous reference 18S ribosomal RNA (18S), real-time quantitative RT-PCR was performed using an GeneAmp 5700 Sequence Detector (PE Biosystems, Foster City, CA) according to the manufacturer's instructions. The derived normalized values are the averages of 4 runs.

Statistics: All data are shown as mean ± SE unless otherwise specified. Data were analyzed by analysis of variance (ANOVA) using the StatView IV program (Brainpower, Calabasas, CA) on a Macintosh G3. Comparisons between group means were performed by Fisher's least significant difference method. A p value of less than 0.05 was considered statistically significant.

### Results:

Animal Data: Angiotensin II infusion was associated with increased systolic blood pressure (SBP) compared with vehicle treated animals (141 ± 2 mmHg versus 202 ± 7 mmHg, vehicle versus angiotensin II, p < 0.001). Mesenteric vessel weight was also increased in angiotensin II-infused rats (52 mg, p < 0.001), compared with control animals receiving vehicle (32 mg, p < 0.001). lmatinib had no effect on systolic blood pressure (205 ± 10 mmHg) but significantly reduced mesenteric weight in animals receiving angiotensin II (42 mg, p < 0.001).

Histology and immunohistochemistry: Histomorphometric analysis revealed a significant increase in the media wall:lumen ratio in angiotensin II-infused rats (Vehicle -> 0.39; angiotensin II-infused rats -> 0.52 , p < 0.001). Treatment with Imatinib significantly reduced the wall:lumen ratio in both groups to levels approaching those in control animals (0.4, p < 0.001). Trichrome stained sections demonstrated both media hypertrophy and expansion of collagenous ECM in the mesenteric vessels of animals receiving angiotensin II-infusion. Imatinib treatment significantly reduced both media hypertrophy and the extent of ECM expansion in angiotensin II-infused.

Gene Expression: Mesenteric vessel TGF-β1 mRNA was increased two-fold in animals receiving angiotensin II infusions when compared with control animals receiving vehicle. In animals receiving Imatinib treatment, a further increase in TGF-β expression was noted when compared with their untreated counterparts also subjected to angiotensin II-infusion. In contrast, PDGF gene expression was not increased in angiotensin II infused animals and was unaffected by treatment with Imatinib.

### Example 2: Capsules with Imatinib (4-[(4-methyl-1-piperazin-1-ylmethyl)-N-[4-methyl-3-[[4-(3-pyridinyl)-2-pyrimidinyl]amino]phenyl]benzamide) methanesulfonate, β-crystal form.

Capsules containing 119.5 mg of the compound named in the title (= SALT I) corresponding to 100 mg of lmatinib (free base) as active substance are prepared in the following composition:

| Composition | |
|---|---|
| SALT I | 119.5 mg |
| Cellulose MK GR | 92 mg |
| Crospovidone XL | 15 mg |
| Aerosil 200 | 2 mg |
| Magnesium stearate | 1.5 mg |
| | 230 mg |

The capsules are prepared by mixing the components and filling the mixture into hard gelatin capsules, size 1.

### Example 3: Capsules with 4-[(4-methyl-1-piperazin-1-ylmethyl)-N-[4-methyl-3-[[4-(3-pyridinyl)-2-pyrimidinyl]amino]phenyl]benzamide methanesulfonate, β-crystal form

Capsules containing 119.5 mg of SALT I corresponding to 100 mg of COMPOUND I (free base) as active substance are prepared in the following composition:

| Composition | |
|---|---|
| Active substance | 119.5 mg |
| Avicel | 200 mg |
| PVPPXL | 15 mg |
| Aerosil | 2 mg |
| Magnesium stearate | 1.5 mg |
| | 338.0 mg |

The capsules are prepared by mixing the components and filling the mixture into hard gelatin capsules, size 1.

This unforeseeable range of properties means that the use of COMPOUNDS OF THE INVENTION is of particular interest for the manufacture of a medicament for the treatment of Ang II-mediated diseases especially angiotensin II induced hypertrophy or angiotensin II induced hypertrophic remodeling in the cardiovascular system and or in the kidney.

This effect can especially be clinically relevant for patients with injuries in relation to hypertension such as described herein.

### Example 2: Capsules with 4-[(4-methyl-1-piperazin-1-ylmethyl)-N-[4-methyl-3-[[4-(3-pyridinyl)-2-pyrimidinyl]amino]phenyl]benzamide methanesulfonate, β-crystal form and valsatran

Capsules containing 100 mg of Imatinib and 80 mg of Valsartan as active substances are prepared in the following composition:

| Composition | |
|---|---|
| lmatinib | 100 mg |
| Valsatran | 80mg |
| Avicel | 200mg |
| PVPPXL | 15 mg |
| Aerosil | 2 mg |
| Magnesium stearate | 1.5 mg |
| | 398.5 mg |

The capsules are prepared by mixing the components and filling the mixture into hard gelatin capsules, size 1.

### Example 3: Capsules with 4-[(4-methyl-1-piperazin-1-ylmethyl)-N-[4-methyl-3-[[4-(3-pyridinyl)-2-pyrimidinyl]amino]phenyl]benzamide methanesulfonate, β-crystal form and valsatran and hydrochlorothiazide.

Capsules containing 100 mg of Imatinib, 80 mg of Valsartan and 12.5 mg of Hydrochlorothiazide as active substances are prepared in the following composition:

| Composition | |
|---|---|
| lmatinib | 100 mg |
| Valsartan | 80mg |
| Hydrochlorothiazide | 12.5mg |
| Avicel | 189mg |
| PVPPXL | 15 mg |
| Aerosil | 2 mg |
| Magnesium stearate | 1.5 mg |
| | 400 mg |

The capsules are prepared by mixing the components and filling the mixture into hard gelatin capsules, size 1.

### Example 4: Capsules with 4-[(4-methyl-1-piperazin-1-ylmethyl)-N-[4-methyl-3-[[4-(3-pyridinyl)-2-pyrimidinyl]amino]phenyl]benzamide methanesulfonate, β-crystal form and valsatran.

| Components | Compostion Per Unit (mg) |
|---|---|
| Valsartan [= active ingredient] | 160.00 |
| Imatinib [= active ingredient] | 80.00 |
| Microcrystalline cellulose | 25.10 |
| Crospovidone | 13.00 |
| Povidone | 12.50 |
| Magnesium stearate | 1.30 |
| Sodium lauryl sulphate | 0.60 |
| Shell | |
| Iron oxide, red (C.I. No. 77491, EC No. E 172) | 0.123 |
| Iron oxide, yellow (C.I. No. 77492, EC No. E 172) | 0.123 |
| Iron oxide, black (C.I. No. 77499, EC No. E 172) | 0.245 |
| Titanium dioxide | 1.540 |
| Gelatin | 74.969 |
| Total tablet mass | 389.50 |

The tablet is manufactured e.g. as follows:
Granulation/Drying: Valsartan and microcrystallin cellulose are spray-granulated in a fluidized bed granulator with a granulating solution consisting of povidone and sodium lauryl sulphate dissolved in purified water. The granulate obtained is dried in a fluidized bed dryer. Milling/Blending: The dried granulate is milled together with crospovidone and magnesium stearate. The mass is then blended in a conical srew type mixer for approximately 10 minutes.
Encapsulation: The empty hard gelatin capsules are filled with the blended bulk granules under controlled temperature and humidity conditions. The filed capsules are dedustee, visually inspected, weight checked and quarantined until by Quality assurance department.

## Claims

1. The use of a PDGF receptor tyrosine kinase inhibitor or pharmaceutically acceptable salt thereof for the manufacture of a medicament for treating an Ang II-mediated disease, wherein the angiotensin II-mediated disease is selected from congestive heart failure, heart failure, cardiac hypertrophy, cardiac remodeling after myocardial infarction, pulmonary congestion and cardiac fibrosis in dilated or in hypertrophic cardiomyopathy, hypertrophic cardiomyopathy, diabetic myopathy, stroke prevention in congestive heart failure, left or right ventricular hypertrophy, hypertrophic medial thickening in arteries and/or in large vessels, mesenteric vasculature hypertrophy, renal hyperfiltration such as after portal renal ablation, proteinuria in chronic renal disease, renal arteriopathy as a consequence of hypertension, Nephrosclerosis or hypertensive nephrosclerosis, mesanglial hypertrophy, endothelial dysfunction with pro-inflammatory and pro-oxidant states.

2. The use of N-phenyl-2-pyrimidine-amine derivatives of formula I, 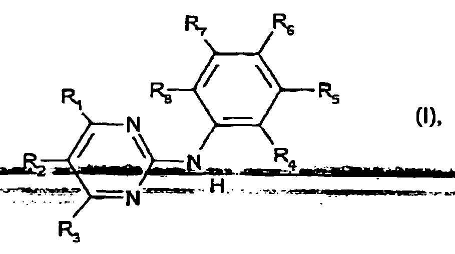 wherein
R₁ is 4-pyrazinyl; 1-methyl-1H-pyrrolyl; amino- or amino-lower alkyl-substituted phenyl, wherein the amino group in each case is free, alkylated or acylated; 1 H-indolyl or 1 H-imidazolyl bonded at a five-membered ring carbon atom: or unsubstituted or lower alkyl-substituted pyridyl bonded at a ring carbon atom and unsubstituted or substituted at the nitrogen atom by oxygen;
R₂ and R₃ are each independently of the other hydrogen or lower alkyl;
one or two of the radicals R₄, R₅, R₈, R₇ and R₈ are each nitro, fluoro-substituted lower alkoxy or a radical of formula II
-N(R₉)-C(=X)-(Y)ₙ-R₁₀ (II).
wherein
R₉ is hydrogen or alkyl (up to and including C₇)
X is oxo, thio, imino, N-lower alkyl-imino, hydroximino or 0-lower alkyl-hydroximino,
Y is oxygen or the group NH,
n is 0 or 1 and
R₁₀ is an aliphatic radical having at least 5 carbon atoms, or an aromatic, aromatic-aliphatic, cycloaliphatic, cycloaliphatic-aliphatic, heterocyclic or heterocyclic-aliphatic radical,
and the remaining radicals R₄, R₅, R₆, R₇ and R₈ are each independently of the others hydrogen, lower alkyl that is unsubstituted or substituted by free or alkylated amino, piperazinyl, piperidinyl, pyrrolidinyl or by morpholinyl, or lower alkanoyl, trifluoromethyl, free, etherified or esterifed hydroxy, free, alkylated or acylated amino or free or esterified carboxy,
or of a salt of such a compound having at least one salt-forming group,
for the manufacture of a medicament for treating Ang II-mediated diseases.

3. Use according to any one of claims 1 or 2, wherein the angiotensin II-mediated disease is selected from endothelial dysfunction with pro-inflammatory and pro-oxidant states.

4. Use according to any one of claims 1 or 2, wherein the angiotensin II-mediated disease is selected from congestive heart failure, heart failure, cardiac hypertrophy, cardiac remodeling after myocardial infarction, pulmonary congestion and cardiac fibrosis in dilated or in hypertrophic cardiomyopathy, hypertrophic cardiomyopathy, diabetic myopathy, stroke prevention in congestive heart failure, left or right ventricular hypertrophy, hypertrophic medial thickening in arteries and/or in large vessels, mesenteric vasculature hypertrophy.

5. Use according to any one of claims 1 or 2, wherein the angiotensin II-mediated disease is renal hyperfiltration such as after portal renal ablation, proteinuria in chronic renal disease, renal arteriopathy as a consequence of hypertension, Nephrosderosis or hypertensive nephrosderosis, mesanglial hypertrophy.

6. Use according to any one of claims 1 or 2, wherein a pharmaceutically acceptable acid addition salt of 4-(4-methylpiperazin-1-ylmethyl)-N-[4-methyl-3-(4-pyridin-3-yl)pyrimidin-2-yl-amino)phenyl]-benzamide of the formula 1 is administered.

7. Use according to any one of claims 1 or 2, wherein a methanesulfonate salt of 4-(4-methylpiperazin-1-ylmethyl)-N-[4-methyl-3-(4-pyridin-3-yl)pyrimidin-2-ylamino)phenyl]-benzamide of the formula I is administered.

8. Use according to any one of claims 1 or 2, wherein a daily dose of 200 to 600 mg of a monomethanesulfonate salt of 4-(4-methylpiperazin-1-ylmethyl)-N-[4-methyl-3-{4-pyridin-3-yl)pyrimidin-2-ylamino)phenyl]-benzamide of the formula I is administered to an adult human.

9. A combination, which comprises (a) N-{5-[4-(4-methyl-piperazino-methyl)-benzoylamido]-2-methylphenyl)-4-(3-pyridyl)-2-pyrimidine-amine, and at least one compound selected from (b) an antihypertensive, an aldosterone antagonist, an aldosterone synthase inhibitor and/or an angiotensin receptor blocker agent wherein the active ingredients are present independently of each other in free form or in the form of a pharmaceutically acceptable salt and optionally at least one pharmaceutically acceptable carrier; for simultaneous, separate or sequential use.

10. Combination according to claim 9 wherein the compound (a) is used in the form of its monomethanesulfonate salt.

11. Combination according to claim 9 or 10, which is a combined, preparation or a pharmaceutical composition.

12. A pharmaceutical composition comprising a quantity which is jointly therapeutically effective against hypertensive-mediated diseases of a combination according to claim 10 or 11 and at least one pharmaceutically acceptable carrier.

13. Use of a combination according to any one of claims 9 to 11 for the delay of progression or treatment of a hypertensive-mediated disease.

14. Use of a combination according to any one of claims 9 to 11 for the preparation of a medicament for the delay of progression or treatment of hypertension induced cardiovascular hypertrophy or cardiovascular hypertrophic remodeling.

15. Use or combination according to any one of claims 9 to 11 wherein there is at least one combination partner (b) selected from the group consisting of valsartan, fluvastatin, atorvastatin, pitavastatin, benzepril, enalapril, amlodipine, especially the besylate thereof, the (+) enantiomer of fadrozole, eplerenone, omapatrilate, Z 13752A, sitaxsentan, especially sitaxsentan sodium, darusentan and hydrochlorothiazide.

16. Use or combination according to any one of claims 9 to 14 wherein the combination partner (b) is Valsartan and/or Hydrochlorothiazide.

17. A commercial package comprising (a) N-{5-[4-(4-methyl-piperazino-methyl)-benzoyl-amido]-2-methylphenyl}-4-(3-pyridyl)-2-pyrimidine-amine and at least one compound selected from (b) an antihypertensive, an aldosterone antagonist, an aldosterone synthase inhibitor and/or an angiotensin receptor blocker agent, together with instructions for simultaneous, separate or sequential use thereof in the delay of progression or treatment of hypertensive-mediated diseases.

18. A combination, which comprises (a) a PDGF receptor tyrosine kinase inhibitor, and (b) Valsartan and optionally Hydrochlorothiazide, wherein the active ingredients are present independently of each other in free form or in the form of a pharmaceutically acceptable salt and optionally at least one pharmaceutically acceptable carrier; for simultaneous, separate or sequential use.

## Patentansprüche

1. Verwendung eines PDGF-Rezeptortyrosinkinasehemmers oder pharmazeutisch annehmbaren Salzes davon zur Herstellung eines Arzneimittels zur Behandlung einer Angiotensin II(Ang II)-vermittelten Erkrankung, wobei die Angiotensin II-vermittelte Erkrankung ausgewählt ist aus kongestiver Herzinsuffizienz, Herzinsuffizienz, Herzhypertrophie, Herzumbau nach Myokardinfarkt, Lungenstauung und Herzfibrose bei dilatativer oder bei hypertropher Kardiomyopathie, hypertropher Kardiomyopathie, diabetischer Myopathie, Schlaganfallprävention bei kongestiver Herzinsuffizienz, links- oder rechtsventrikulärer Hypertrophie, hypertropher medialer Verdickung in Arterien und/oder in großen Gefäßen, mesenteraler vaskulärer Hypertrophie, renaler Hyperfiltration, wie nach portaler Nierenentfernung, Proteinurie bei chronischer Nierenerkrankung, renaler Arteriopathie als einer Folge von Hypertonie, Nephrosklerose oder hypertensiver Nephrosklerose, mesangialer Hypertrophie, Endotheldysfunktion mit proinflammatorischen und prooxidativen Zuständen.

2. Verwendung von N-Phenyl-2-pyrimidinaminderivaten der Formel I 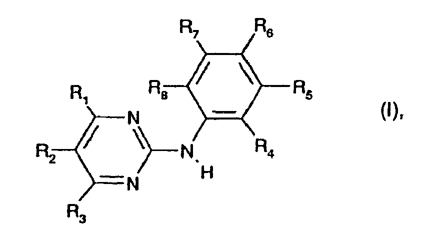 wobei
R₁ steht für 4-Pyrazinyl; 1-Methyl-1 H-pyrrolyl; Amino- oder Aminoniederalkyl-substituiertes Phenyl, wobei die Aminogruppe in jedem Fall frei, alkyliert oder acyliert ist; 1 H-Indolyl oder 1 H-Imidazolyl, das an ein fünfgiiedriges Ringkohlenstoffatom gebunden ist; oder unsubstituiertes oder Niederalkyl-substituiertes Pyridyl, das an ein Ringkohlenstoffatom gebunden und unsubstituiert oder an dem Stickstoffatom durch Sauerstoff substituiert ist;
R₂ und R₃ jeweils unabhängig voneinander für Wasserstoff oder Niederalkyl stehen;
einer oder zwei der Reste R₄, R₅, R₆, R₇ und R₈ jeweils für Nitro, Fluor-substituiertes Niederalkoxy oder einen Rest der Formel II stehen
-N(R₉)-C(=X)-(Y)ₙ-R₁₀ (II),
wobei
R₉ für Wasserstoff oder Alkyl (bis zu und einschließlich C₇) steht,
X für Oxo, Thio, Imino, N-Niederalkylimino, Hydroxyimino oder O-Niederalkylhydroxyimino steht,
Y für Sauerstoff oder die Gruppe NH steht,
n für 0 oder 1 steht, und
R₁₀ für einen aliphatischen Rest mit mindestens 5 Kohlenstoffatomen oder für einen aromatischen, aromatisch-aliphatischen, cycloaliphatischen, cycloaliphatisch-aliphatischen, heterocyclischen oder heterocyclischaliphatischen Rest steht,
und die übrigen Reste R₄, R₅, R₆, R₇ und R₈ jeweils unabhängig von den anderen stehen für Wasserstoff, Niederalkyl, das unsubstituiert oder substituiert ist durch freies oder alkyliertes Amino, Piperazinyl, Piperidinyl, Pyrrolidinyl oder durch Morpholinyl, oder Niederalkanoyl, Trifluormethyl, freies, verethertes oder verestertes Hydroxy, freies, alkyliertes oder acyliertes Amino oder freies oder verestertes Carboxy,
oder von einem Salz von einer solchen Verbindung, die mindestens eine salzbildende Gruppe aufweist, zur Herstellung eines Arzneimittels zur Behandlung Ang II-vermittelter Erkrankungen.

3. Verwendung nach einem der Ansprüche 1 oder 2, wobei die Angiotensin II-vermittelte Erkrankung ausgewählt ist aus Endotheldysfunktion mit proinflammatorischen und prooxidativen Zuständen.

4. Verwendung nach einem der Ansprüche 1 oder 2, wobei die Angiotensin II-vermittelte Erkrankung ausgewählt ist aus kongestiver Herzinsuffizienz, Herzinsuffizienz, Herzhypertrophie, Herzumbau nach Myokardinfarkt, Lungenstauung und Herzfibrose bei dilatativer oder bei hypertropher Kardiomyopathie, hypertropher Kardiomyopathie, diabetischer Myopathie, Schlaganfallprävention bei kongestiver Herzinsuffizienz, links- oder rechtsventrikulärer Hypertrophie, hypertropher medialer Verdickung in Arterien und/oder in gro-ßen Gefäßen, mesenteraler vaskulärer Hypertrophie.

5. Verwendung nach einem der Ansprüche 1 oder 2, wobei die Angiotensin II-vermittelte Erkrankung für renale Hyperfiltration, wie nach portaler Nierenentfernung, Proteinurie bei chronischer Nierenerkrankung, renale Arteriopathie als eine Folge von Hypertonie, Nephrosklerose oder hypertensive Nephrosklerose, mesangiale Hypertrophie steht.

6. Verwendung nach einem der Ansprüche 1 oder 2, wobei ein pharmazeutisch annehmbares Säureadditionssalz von 4-(4-Methylpiperazin-1-ylmethyl)-N-[4-methyl-3-(4-pyridin-3-yl)pyrimidin-2-ylamino)phenyl]-benzamid der Formel I verabreicht wird.

7. Verwendung nach einem der Ansprüche 1 oder 2, wobei ein Methansulfonatsalz von 4-(4-Methylpiperazin-1-ylmethyl)-N-[4-methyl-3-(4-pyridin-3-yl)pyrimidin-2-ylamino)phenyl]-benzamid der Formel I verabreicht wird.

8. Verwendung nach einem der Ansprüche 1 oder 2, wobei eine tägliche Dosis von 200 bis 600 mg eines Monomethansulfonatsalzes von 4-(4-Methylpiperazin-1-ylmethyl)-N-[4-methyl-3-(4-pyridin-3-yl)-pyrimidin-2-ylamino)phenyl]-benzamid der Formel I einem erwachsenen Menschen verabreicht wird.

9. Kombination, die umfasst (a) N-{5-[4-(4-Methyl-piperazino-methyl)-benzoylamido]-2-methylphenyl}-4-(3-pyridyl)-2-pyrimidin-amin und mindestens eine Verbindung, die ausgewählt ist aus (b) einem Antihypertensivum, einem Aldosteronantagonisten, einem Aldosteronsynthase-Hemmer und/oder einem Angiotensinrezeptorblocker, wobei die wirksamen Inhaltsstoffe unabhängig voneinander in freier Form oder in der Form eines pharmazeutisch annehmbaren Salzes vorliegen, und gegebenenfalls mindestens einen pharmazeutisch annehmbaren Träger; zur gleichzeitigen, getrennten oder sequenziellen Verwendung.

10. Kombination nach Anspruch 9, wobei die Verbindung (a) in der Form ihres Monomethansulfonatsalzes verwendet wird.

11. Kombination nach Anspruch 9 oder 10, die eine kombinierte Zubereitung oder eine pharmazeutische Zusammensetzung ist.

12. Pharmazeutische Zusammensetzung, umfassend eine Menge, die gemeinsam therapeutisch wirksam ist gegen hypertensiv vermittelte Erkrankungen, von einer Kombination gemäß Anspruch 10 oder 11 und mindestens einen pharmazeutisch annehmbaren Träger.

13. Verwendung einer Kombination nach einem der Ansprüche 9 bis 11 zur Verzögerung des Fortschreitens oder Behandlung einer hypertensiv vermittelten Erkrankung.

14. Verwendung einer Kombination nach einem der Ansprüche 9 bis 11 zur Herstellung eines Arzneimittels für die Verzögerung des Fortschreitens oder Behandlung von Hypertonie, die induziert wird durch kardiovaskuläre Hypertrophie oder kardiovaskulären hypertrophen Umbau.

15. Verwendung oder Kombination nach einem der Ansprüche 9 bis 11, wobei es mindestens einen Kombinationspartner (b) gibt, der ausgewählt ist aus der Gruppe, bestehend aus Valsartan, Fluvastatin, Atorvastatin, Pitavastatin, Benzepril, Enalapril, Amlodipin, insbesondere das Besilat davon, das (+)-Enantiomer von Fadrozol, Eplerenon, Omapatrilat, Z 13752A, Sitaxsentan, insbesondere Sitaxsentan-Natrium, Darusentan und Hydrochlorthiazid.

16. Verwendung oder Kombination nach einem der Ansprüche 9 bis 14, wobei der Kombinationspartner (b) für Valsartan und/oder Hydrochlorthiazid steht.

17. Kommerzielle Verpackung, umfassend (a) N-{5-[4-(4-Methyl-piperazino-methyl)-benzoylamido]-2-methylphenyl}-4-(3-pyridyl)-2-pyrimidin-amin und mindestens eine Verbindung, die ausgewählt ist aus (b) einem Antihypertensivum, einem Aldosteronantagonisten, einem Aldosteronsynthase-Hemmer und/oder einem Angiotensinrezeptorblocker, zusammen mit Anleitungen zur simultanen, getrennten oder sequenziellen Verwendung davon bei der Verzögerung des Fortschreitens oder Behandlung von hypertensiv vermittelten Erkrankungen.

18. Kombination, die umfasst (a) einen PDGF-Rezeptortyrosinkinasehemmer und (b) Valsartan und gegebenenfalls Hydrochlorthiazid, wobei die wirksamen Inhaltsstoffe unabhängig voneinander in freier Form oder in der Form eines pharmazeutisch annehmbaren Salzes vorliegen, und gegebenenfalls mindestens einen pharmazeutisch annehmbaren Träger; zur simultanen, getrennten oder sequenziellen Verwendung.

## Revendications

1. Utilisation d'un inhibiteur de la tyrosine-kinase du récepteur de PDGF ou d'un sel pharmaceutiquement acceptable de celui-ci pour la fabrication d'un médicament destiné au traitement d'une maladie à médiation par Ang II, où la maladie à médiation par l'angiotensine II est choisie parmi l'insuffisance cardiaque congestive, l'insuffisance cardiaque, l'hypertrophie cardiaque, le remodelage cardiaque après un infarctus du myocarde, la congestion pulmonaire et la fibrose cardiaque dans le cas d'une cardiomyopathie dilatée ou obstructive, la cardiomyopathie obstructive, la myopathie diabétique, la prévention de l'ictus dans le cas d'une insuffisance cardiaque congestive, l'hypertrophie ventriculaire gauche ou droite, l'épaississement médian hypertrophique des artères et/ou des gros vaisseaux, l'hypertrophie du système vasculaire mésentérique, l'hyperfiltration rénale comme à la suite d'une ablation rénale porte, la protéinurie lors d'une maladie rénale chronique, l'artériopathie rénale suite à une hypertension, la néphrosclérose ou la néphrosclérose hypertensive, l'hypertrophie mésangliale, le dysfonctionnement endothélial avec des états pro-inflammatoires et pro-oxydants.

2. Utilisation de dérivés de N-phényl-2-pyrimidine-amine de formule I, 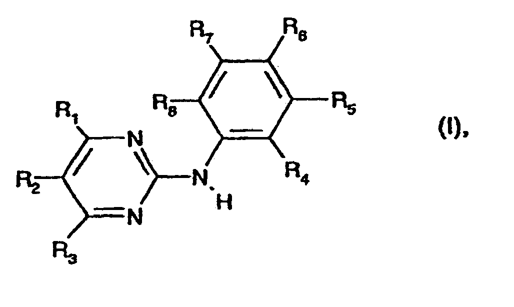 dans laquelle
R₁ est un radical 4-pyrazinyle; 1-méthyl-1H-pyrrolyle; phényle à substitution amino ou amino-alkyle inférieur, où le groupe amino est, dans chaque cas, libre, alkylé ou acylé; 1H-indolyle ou 1H-imidazolyle lié à un atome de carbone de cycle à cinq chaînons; ou pyridyle non substitué ou à substitution alkyle inférieur lié à un atome de carbone de cycle et non substitué ou substitué au niveau de l'atome d'azote par un oxygène;
R₂ et R₃ représentent chacun, indépendamment l'un de l'autre, un atome d'hydrogène ou un groupe alkyle inférieur; un ou deux des radicaux R₄, R₅, R₆, R₇ et R₈ représentent chacun un groupe nitro, alcoxy inférieur fluoré ou un radical de formule II
-N(R₉)-C(=X)-(Y)ₙ-R₁₀ (II)
dans laquelle
R₉ est un atome d'hydrogène ou un groupe alkyle (allant jusqu'à C₇ compris)
X est un groupe oxo, thio, imino, N-alkyl(inférieur)-imino, hydroxyimino ou O-alkyl(inférieur)-hydroxyimino,
Y est un atome d'oxygène ou le groupe NH,
n vaut 0 ou 1 et
R₁₀ est un radical aliphatique ayant au moins 5 atomes de carbone, ou un radical aromatique, aromatique-aliphatique, cycloaliphatique, cycloaliphatique-aliphatique, hétérocyclique ou hétérocyclique-aliphatique,
et les radicaux R₄, R₅, R₆, R₇ et R₈ restants représentent chacun,
indépendamment les uns des autres, un atome d'hydrogène, un groupe alkyle inférieur qui est non substitué ou substitué par un groupe amino libre ou alkylé, pipérazinyle, pipéridinyle, pyrrolidinyle ou par un groupe morpholinyle, ou un groupe alcanoyle inférieur, trifluorométhyle, hydroxy libre, éthérifié ou estérifié, amino libre, alkylé ou acylé ou carboxy libre ou estérifié,
ou d'un sel d'un composé de ce type ayant au moins un groupe formant un sel,
pour la fabrication d'un médicament destiné au traitement des maladies à médiation par Ang II.

3. Utilisation selon l'une quelconque des revendications 1 et 2, où la maladie à médiation par l'angiotensine II est choisie parmi un dysfonctionnement endothélial avec des états pro-inflammatoires et pro-oxydants.

4. Utilisation selon l'une quelconque des revendications 1 et 2, où la maladie à médiation par l'angiotensine II est choisie parmi l'insuffisance cardiaque congestive, l'insuffisance cardiaque, l'hypertrophie cardiaque, le remodelage cardiaque après un infarctus du myocarde, la congestion pulmonaire et la fibrose cardiaque dans le cas d'une cardiomyopathie dilatée ou obstructive, la cardiomyopathie obstructive, la myopathie diabétique, la prévention de l'ictus dans le cas d'une insuffisance cardiaque congestive, l'hypertrophie ventriculaire gauche ou droite, l'épaississement médian hypertrophique des artères et/ou des gros vaisseaux, l'hypertrophie du système vasculaire mésentérique.

5. Utilisation selon l'une quelconque des revendications 1 et 2, où la maladie à médiation par l'angiotensine II est l'hyperfiltration rénale comme à la suite d'une ablation rénale porte, la protéinurie lors d'une maladie rénale chronique, l'artériopathie rénale suite à une hypertension, la néphrosclérose ou la néphrosclérose hypertensive, l'hypertrophie mésangliale.

6. Utilisation selon l'une quelconque des revendications 1 et 2, où un sel d'addition d'acide pharmaceutiquement acceptable du 4-(4-méthylpipérazin-1-ylméthyl)-N-[4-méthyl-3-(4-pyridin-3-yl)pyrimidin-2-ylamino)phényl]-benzamide de formule I est administré.

7. Utilisation selon l'une quelconque des revendications 1 et 2, où un sel de méthanesulfonate de 4-(4-méthylpipérazin-1-ylméthyl)-N-[4-méthyl-3-(4-pyridin-3-yl)pyrimidin-2-ylamino)phényl]-benzamide de formule I est administré.

8. Utilisation selon l'une quelconque des revendications 1 et 2, où une dose journalière de 200 à 600 mg d'un sel de monométhanesulfonate de 4-(4-méthylpipérazin-1-ylméthyl)-N-[4-méthyl-3-(4-pyridin-3-yl)pyrimidin-2-ylamino)phényl]-benzamide de formule I est administrée à un humain adulte.

9. Combinaison qui comprend (a) la N-{5-[4-(4-méthyl-pipérazino-méthyl)benzoylamido]-2-méthylphényl}-4-(3-pyridyl)-2-pyrimidine-amine, et au moins un composé choisi parmi (b) un antihypertenseur, un antagoniste d'aldostérone, un inhibiteur de l'aldostérone-synthase et/ou un agent bloquant des récepteurs d'angiotensine, dans laquelle les principes actifs sont présents, indépendamment les uns des autres, sous forme libre ou sous la forme d'un sel pharmaceutiquement acceptable, et éventuellement au moins un véhicule pharmaceutiquement acceptable, pour un usage simultané, séparé ou séquentiel.

10. Combinaison selon la revendication 9, dans laquelle le composé (a) est utilisé sous la forme de son sel de monométhanesulfonate.

11. Combinaison selon la revendication 9 ou 10, qui est une préparation combinée ou une composition pharmaceutique.

12. Composition pharmaceutique comprenant une quantité, qui est conjointement thérapeutiquement efficace contre les maladies d'origine hypertensive, d'une combinaison selon la revendication 10 ou 11, et au moins un véhicule pharmaceutiquement acceptable.

13. Utilisation d'une combinaison selon l'une quelconque des revendications 9 à 11 pour retarder la progression ou le traitement d'une maladie d'origine hypertensive.

14. Utilisation d'une combinaison selon l'une quelconque des revendications 9 à 11 pour la préparation d'un médicament pour retarder la progression ou le traitement d'une hypertrophie cardiovasculaire induite par l'hypertension ou d'un remodelage hypertrophique cardiovasculaire.

15. Utilisation ou combinaison selon l'une quelconque des revendications 9 à 11, où il y a au moins un partenaire de combinaison (b) choisi dans le groupe constitué par le valsartan, la fluvastatine, l'atorvastatine, la pitavastatine, le benzépril, l'énalapril, l'amlodipine, en particulier son bésylate, l'énantiomère (+) du fadrozole, l'éplérénone, l'omapatrilate, le Z 13752A, le sitaxsentan, en particulier le sitaxsentan sodique, le darusentan et l'hydrochlorothiazide.

16. Utilisation ou combinaison selon l'une quelconque des revendications 9 à 14, où le partenaire de combinaison (b) est le Valsartan et/ou l'Hydrochlorothiazide.

17. Conditionnement commercial comprenant (a) la N-{5-[4-(4-méthyl-pipérazino-méthyl)benzoylamido]-2-méthylphényl}-4-(3-pyridyl)-2-pyrimidine-amine, et au moins un composé choisi parmi (b) un antihypertenseur, un antagoniste d'aldostérone, un inhibiteur de l'aldostérone-synthase et/ou un agent bloquant des récepteurs d'angiotensine, conjointement avec les instructions pour leur usage simultané, séparé ou séquentiel pour retarder la progression ou le traitement des maladies d'origine hypertensive.

18. Combinaison qui comprend (a) un inhibiteur de la tyrosine-kinase du récepteur de PDGF, et (b) le Valsartan et éventuellement l'Hydrochlorothiazide, dans laquelle les principes actifs sont présents, indépendamment les uns des autres, sous forme libre ou sous la forme d'un sel pharmaceutiquement acceptable, et éventuellement au moins un véhicule pharmaceutiquement acceptable, pour un usage simultané, séparé ou séquentiel.
